# EUROPÄISCHE PATENTSCHRIFT

(11) **EP 3 755 669 B1**
(45) Veröffentlichungstag und Bekanntmachung des Hinweises auf die Patenterteilung: **16.08.2023**
(21) Anmeldenummer: 19704769.9
(22) Anmeldetag: 06.02.2019
(51) Int. Cl.: C03C 4/20, A61J 1/06, A61J 1/14, C03C 23/00

(54) **GLASFLÄSCHCHEN MIT GERINGER MIGRATIONSLAST**
SMALL GLASS BOTTLE HAVING LOWER MIGRATION LOAD
FLACONS EN VERRE À CHARGE DE MIGRATION RÉDUITE

(30) Priorität: 23.02.2018 DE 102018104163
(43) Veröffentlichungstag der Anmeldung: 30.12.2020
(73) Patentinhaber: SCHOTT Pharma AG & Co. KGaA, 55122 Mainz (DE)
(72) Erfinder: FROST, Robert, 9035 Grub AR (CH); ROTHHAAR, Uwe, 67134 Birkenheide (DE); BUSCKE, Florence, 55116 Mainz (DE); HLADIK, Bernhard, 55232 Alzey (DE)
(74) Vertreter: Blumbach · Zinngrebe Patentanwälte PartG mbB
(86) Internationale Anmeldenummer: PCT/EP2019/052925
(87) Internationale Veröffentlichungsnummer: WO 2019/162094

(56) Entgegenhaltungen:
- EP-A1- 2 119 512
- US-A1- 2010 255 229
- DATABASE WPI Week 198834 Thomson Scientific, London, GB; AN 1988-238070 XP002791273, -& JP S63 170233 A (NICHIDEN RIKAGLASS) 14. Juli 1988 (1988-07-14)
- DATABASE WPI Week 201320 Thomson Scientific, London, GB; AN 2013-D26655 XP002791274, -& JP 2013 047111 A (ISHIZUKA GLASS KK) 7. März 2013 (2013-03-07)

## Beschreibung

### Gebiet der Erfindung

Im Allgemeinen betrifft die Erfindung Glasfläschchen zur Lagerung von pharmazeutischen Wirkstoffen. Im Speziellen betrifft die Erfindung Glasfläschchen, die auch zur Lagerung von kleinen Mengen von pharmazeutischen Wirkstoffen verwendet werden können, wobei die Wirksamkeit der pharmazeutischen Wirkstoffe sich während der Lagerung im Glasfläschchen nicht oder nur in sehr geringem Maße ändert.

### Stand der Technik

Einige pharmazeutische Wirkstoffe wie beispielsweise therapeutische Proteine, biotechnologisch hergestellte Wirkstoffe wie beispielsweise monoklonale Antikörper und Vakzine werden häufig in sehr kleinen Mengen verabreicht. Die entsprechenden Füllvolumen sind hierbei in der Regel signifikant geringer als die Nennvolumina der kleinsten auf dem Markt verfügbaren Primärpackmittel aus Neutralglas.

Unter dem Nennvolumen wird hierbei das Produktvolumen, beispielsweise das Volumen einer pharmazeutischen Wirkstoffzubereitung verstanden, die in dem entsprechenden Packmittel enthalten sein sollte, sofern dieses komplett befüllt ist. Hiervon zu unterscheiden ist das sog. Randvollvolumen, welches einer Füllmenge des entsprechenden Packmittels bis zu dessen Rand entspricht. In der Regel ist das Nennvolumen kleiner als das Randvollvolumen. Häufig beträgt das Randvollvolumen das 1,5 bis 2,5-fache des Nennvolumens.

Als verfügbare Packmittel kommen grundsätzlich Spritzen, Karpulen und sog. Vials, d,h, Glasfläschchen in Frage. Spritzen und Karpulen weisen jedoch an ihrer Glasinnenoberfläche eine silikonhaltige Gleitschicht auf, um dem Gummistopfen das Gleiten bei der Entleerung zu ermöglichen. Insbesondere die oben genannten pharmazeutischen Wirkstoffe erleiden jedoch eine deaktivierende Wechselwirkung mit der Gleitschicht, so dass Spritzen und Karpulen für pharmazeutische Zubereitungen mit diesen Wirkstoffen nicht als Primärpackmittel verwendet werden können.

Daher werden sog. Neutralglasvials bzw. -fläschchen als Primärpackmittel verwendet. Sie werden mit Kappen verschlossen, welche keine beeinträchtigende Stoffabgabe aufweisen und werden stehend gelagert. Insbesondere werden Glasfläschchen aus sog. Neutralglas vom Typ I (gemäß EP 3.2.1 oder USP 660) verwendet, die als Röhrenglasfläschchen durch einen Heißumformungsprozess hergestellt werden.

Wird der pharmazeutische Wirkstoff beispielsweise in einer geeigneten Pufferlösung in das Vial gefüllt, kann es sein, dass ein Teil der in den Vials abgepackten pharmazeutischen Zubereitungen über die gesamte Lagerdauer stabil ist während ein anderer Teil der Zubereitungen durch eine zu hohe Migrationslast eine verminderte Wirksamkeit aufweist.

Die unterschiedliche Migrationslast innerhalb einer Charge hat auch zur Folge, dass Lagerstudien und Stichproben nicht aussagekräftig sind.

Um die oben genannten Nachteile zu vermeiden, ist aus dem Stand der Technik die Verwendung von Neutralglasvials vom Typ 1 bekannt, die in Folge einer Ammoniumsulfatbehandlung eine stark reduzierte und weitgehend einheitliche Abgabe von Alkaliionen aufweisen. Durch die Behandlung mit Ammoniumsulfat werden den oberflächennahen Glasschichten bis ca. 50 µm mobile, d.h. nicht fest in das Glas eingebundene Alkaliionen entzogen. Jedoch kann eine Ammoniumsulfatbehandlung eine Migration von Nicht-Alkali-Bestandteilen und einer Diffusion von Glasbestandteilen an die Glasoberfläche und eine anschließende Migration in das Füllgut während der Lagerungszeit nicht verhindern. Ein weiterer Nachteil der Ammoniumsulfatbehandlung besteht in einer Schädigung der Glasoberfläche durch die beim Prozess herrschenden hohen Temperaturen und die damit einhergehende Verminderung der chemischen Beständigkeit

Eine weitere im Stand der Technik beschriebene Möglichkeit die Auslaugung der oben beschriebenen Glasbestandteile zu verringern besteht in einer Innenbeschichtung der Glasfläschchen mit einer SiO₂-Beschichtung. Neben den hohen Herstellungskosten ist jedoch auch die begrenzte Stabilität der Quarzglasbeschichtungen bei pH-Werten im alkalischen Bereich nachteilig. Die oben genannten Probleme verschärfen sich noch, wenn das Glasfläschchen nicht voll befüllt wird, da das Verhältnis von benetzter Oberfläche zu Füllvolumen ansteigt. Die Dokumente JP S63 170233 A und US 2014/0224693 A1 beschreiben Glasfläschchen für pharmazeutische Wirkstoffe.

### Aufgabe der Erfindung

Es ist daher Aufgabe der Erfindung, Glasfläschchen bereitzustellen, welche als Pharmapackmittel auch für sehr geringe Füllmengen verwendet werden können und welche die oben beschriebenen Nachteile nicht aufweisen. Insbesondere soll ein Pharmapackmittel für pharmazeutische Zubereitungen bereitgestellt werden, welches eine Wirkungsbeeinträchtigung des Füllgutes innerhalb der geplanten Lagerungsdauer zuverlässig ausschließt und zudem eine verlässliche Homogenität der chemischen Eigenschaften über die gesamte jeweilige Herstellungscharge aufweist.

### Kurzbeschreibung der Erfindung

Die Aufgabe der Erfindung wird bereits durch den Gegenstand der unabhängigen Ansprüche gelöst. Vorteilhafte Ausgestaltungen und Weiterbildungen der Erfindung sind dabei Gegenstand der Unteransprüche.

Die Erfindung sieht zur Lösung der Aufgabe ein Glasfläschchen vor, welches bereits ohne Innenbeschichtungen durch die Ausgestaltung des Glases an der Innenseite die Migration von unerwünschten Verunreinigungen reduziert und entsprechend als Pharmapackmittel verwendet werden kann.

Ein Aspekt der vorliegenden Erfindung ist die Verwendung eines Glasfläschchens mit einem Gesamtvolumen < 4,5 ml aus einem Mehrkomponentenglas zur Befüllung mit einer flüssigen pharmazeutischen Zubereitung (9) bis zu einem Befüllungsgrad von maximal 0,25. Unter dem Gesamtvolumen < 4,5 ml wird hierbei das Randvollvolumen des Glasfläschchens verstanden. Der Befüllungsgrad des Glasfläschchens ergibt sich demnach aus dem Verhältnis des Füllvolumens, d.h. des Volumens der pharmazeutischen Zubereitung im Glasfläschchen und dem Randvollvolumen des Glasfläschchens. Unter einem Mehrkomponentenglas wird im Sinne der Erfindung ein Glas verstanden, welches neben SiO₂ zumindest einen weiteren Glasbestandteil aufweist. Unter einem Bestandteil im Sinne der Erfindung ist insbesondere ein chemisches Element zu verstehen, das im Glas mit mindestens 1% des Gesamtgewichts enthalten ist.

Bei der Herstellung von Glasfläschchen aus einem Glasrohr wird das Glasrohr durch Heißumformung so bearbeitet, dass ein Boden ausgebildet wird. Im Herstellungsprozess erfahren dabei die Glasbereiche, die den Boden sowie den bodennahen Randbereich bilden, die stärkste Temperaturerhöhung und damit verbunden die stärkste Veränderung der chemischen Zusammensetzung. So weisen beispielsweise entsprechende Borosilikatgläser nach der Umformung im bodennahen Wandbereich eine oberflächennahe Konzentrationsüberhöhung von Alkaliionen und Borionen auf. Daher werden in diesen Bereichen bei herkömmlichen Glasfläschchen quantitativ mehr Glasbestandteile beim Kontakt mit Flüssigkeiten abgegeben als in den unverformten zylindrischen Wandbereichen. Die Abgabe der Glasbestandteile an die Flüssigkeit wird auch als Auslösen oder Auslaugen bezeichnet, wobei nicht nur Alkaliionen sondern auch weitere Glasbestandteile wie Bor, Aluminium oder Silizium ausgelöst werden können. Gläser bzw. Glasbereiche mit einer hohen Abgabe an Glasbestandteilen an die Flüssigkeit werden entsprechend als auslöse- oder auslaugfreundlich bezeichnet. Insbesondere der bodennahe Wandbereich gibt bei Kontakt mit einer Flüssigkeit quantitativ mehr Glasbestandteile an die Flüssigkeit ab als die unverformten Bereiche. Die ausgelösten Glasbestandteile können dabei mit dem Inhalt des Vials bzw. des Glasfläschchens wechselwirken und so beispielsweise die Stabilität und Wirksamkeit von pharmazeutischen Zubereitungen erheblich verringern.

Bei Borosilikatgläsern handelt es sich bei den migrierten Bestandteilen vorrangig um die Alkalien Na, K und Ca sowie die Glasbestandteile B, AI und Si. Migrieren Na, K und Ca in das Füllgut, so bewirken sie eine Verschiebung des pH-Wertes in den alkalischen Bereich. Durch Verwendung von geeigneten Pufferlösungen kann dieser pH-Wertverschiebung bis zu einem gewissen Punkt entgegengewirkt werden, jedoch nimmt die pH-Wertverschiebung mit der Lagerdauer zu und ist das häufigste Problem bei Produkten mit kleinen Abfüllmengen. So wird bei den aus dem Stand der Technik bekannten Glasfläschchen in etwa der Hälfte der Fälle keine ausreichende Pufferung über die gesamte Lagerdauer erreicht.

Hinzu kommt, dass die Heißumformungsprozesse bei der Herstellung herkömmlicher, d.h. aus dem Stand der Technik bekannter Vials häufig lediglich in Hinblick auf die geometrische Spezifikation der Vials reproduzierbar sind. Somit variiert insbesondere die chemische Zusammensetzung der Glasoberfläche in den bodennahen Bereichen und somit auch die Migration von Glasbestandteilen in eine abgefüllte pharmazeutische Zubereitung qualitativ wie auch quantitativ. Der quantitative Anteil der ausgelösten Bestandteile bezogen auf das Volumen der Flüssigkeit wird als Migrationslast bezeichnet. Selbst Vials einer Charge können sich in Bezug auf die Migrationslast unterscheiden.

Die Innenwand des erfindungsgemäßen Glasfläschchens weist dabei eine gegenüber dem Stand der Technik erhöhte Hydrolysebeständigkeit, d.h. eine chemische Beständigkeit gegen ein Auslösen zumindest eines der Bestandteile des Mehrkomponentenglases auf.

Die chemische Beständigkeit führt dabei dazu, dass bei Auslösen von Glasbestandteilen des Glases mit einer Flüssigkeit als Auslöse- bzw. Auslaugmedium die Konzentration zumindest eines ausgelösten Bestandteiles bei einem Füllvolumen von 0,5 ml und der Konzentration bei einem Füllvolumen von 2 ml bei maximal 3 und das Verhältnis zwischen der Konzentration bei einem Füllvolumen von 1 ml und der Konzentration bei einem Füllvolumen von 2 ml maximal bei 2 liegt. Hierbei wird das Glasfläschchen mit der jeweiligen Menge der als Auslösemedium verwendeten Flüssigkeit versetzt und das so befüllte Glasfläschchen bei einer Temperatur von 40°C für einen Zeitraum von 24 Wochen stehend gelagert. Nach dieser Lagerungszeit wird die Konzentration des oder der ausgelösten Glasbestandteile im Auslösemedium bestimmt. Zur quantitativen Bestimmung der Konzentrationen der in Lösung übergegangene Glaselemente wurden HR-ICP-MS (High Resolution Inductively Coupled Plasma Mass Spectrometry) und ICP-OES (Inductively Coupled Plasma - Optical Emission Spectroscopy) Analysen durchgeführt.

Somit kann die Aufgabe der Erfindung durch die Verwendung eines Glasfläschchens mit einem Gesamtvolumen < 4,5 ml aus einem Mehrkomponentenglas gelöst werden, wobei die Innenwand des Glasfläschchens eine chemische Beständigkeit gegen ein Auslösen zumindest eines der Bestandteile des Mehrkomponentenglases aufweist, wobei
bei Auslösen des Glasfläschchens mit einer wässrigen Flüssigkeit mit einem pH-Wert im Bereich von 5 bis 9 bei einer Temperatur von 40°C über einen Zeitraum von 24 Wochen bei stehender Lagerung des Glasfläschchens das Verhältnis der Konzentration zumindest eines ausgelaugten Bestandteiles bei einem Füllvolumen von 0,5 ml und der Konzentration bei einem Füllvolumen von 2 ml maximal 3 und das Verhältnis zwischen der Konzentration bei einem Füllvolumen von 1 ml und der Konzentration bei einem Füllvolumen von 2 ml maximal 2 liegt, zur Befüllung mit einer flüssigen pharmazeutischen Zubereitung (4) bis zu einem Befüllungsgrad von maximal 0,25. Insbesondere liegt der Wasseranteil der Flüssigkeit bei zumindest 80 Vol.-%.

Gemäß einer Ausführungsform der Erfindung beträgt das Verhältnis zwischen der Konzentration des ausgelösten Glasbestandteiles bei einem Füllvolumen von 0,5 ml und der Konzentration bei einem Füllvolumen von 2 ml maximal 2,5, besonders bevorzugt maximal 1,5 und das Verhältnis zwischen der Konzentration bei einem Füllvolumen von 1 ml und der Konzentration bei einem Füllvolumen von 2 ml maximal 1,5. Bevorzugt liegt das Verhältnis zwischen der Konzentration bei einem Füllvolumen von 1 ml und der Konzentration bei einem Füllvolumen von 2 ml im Bereich von 1 bis 1,8, bevorzugt von 1 bis 1,5.

Bei einem Füllvolumen von 0,5 ml bedeckt die als Auslösemedium eingesetzte Flüssigkeit, im Folgenden auch nur als "Flüssigkeit" bezeichnet, vorwiegend den Boden und den bodennahen Wandbereich des Glasfläschchens und somit die Bereiche des Glasfläschchens, die am meisten zur Migrationslast in der Flüssigkeit beitragen. Das Auslöseverhalten bei einem Füllvolumen von 0,5 ml lässt daher Rückschlüsse auf die Migrationslast bei einem geringen Befüllungsgrad zu.

Bei einem Füllvolumen von 2 ml werden dagegen auch die Bereiche der Glasinnenwand von der Flüssigkeit benetzt, die durch den Umformprozess nicht oder nur wenig in ihrer chemischen Zusammensetzung beeinflusst wurden und somit nicht oder nur in sehr geringem Maße zur Migrationslast der Flüssigkeit im Vial beitragen. Mit dem Füllvolumen von 2 ml wird somit das Auslöseverhalten bei einem hohen Befüllungsgrad erhalten.

Durch das Verhältnis zwischen den Konzentrationen eines ausgelösten Bestandteils bei einem Füllvolumen von 0,5 ml und einem Füllvolumen von 2 ml wird somit deutlich, in wie weit die Migrationslast in den bodennahen Bereichen im Vergleich zu den unverformten Wandbereichen erhöht ist. Der quantitative Anteil der ausgelösten Bestandteile bezogen auf die von der Flüssigkeit benetzten Fläche der Glaswand wird dabei als Auslösestärke bezeichnet. Die für ein Füllvolumen ermittelte Konzentration eines ausgelösten Bestandteils entspricht definitionsgemäß der Auslösungsstärke bezogen auf das Füllvolumen und multipliziert mit der durch die Flüssigkeit benetzten Fläche. Damit entspricht das Verhältnis der Konzentrationen eines ausgelösten Bestandteils dem Verhältnis der Auslösestärken multipliziert zum einen mit dem Verhältnis der benetzten Flächen und zum anderen multipliziert mit dem Umkehrwert des Verhältnisses der Füllvolumen.

Für ein handelsübliches Röhrenglas-Vial mit einem Nennvolumen von 2 ml und einem typischen Innendurchmesser von ca. 14 mm ergibt sich beispielsweise das Verhältnis der benetzten Fläche bei einem Füllvolumen von 0,5 ml zu der benetzten Fläche bei einem Füllvolumen von 2 ml zu einem Wert von ca. 0,4. Für ein solches Vial bedeutet daher ein Verhältnis der Konzentrationen eines ausgelösten Bestandteils beim Füllvolumen von 0,5 ml und einem Füllvolumen von 2 ml größer 1,6 (0,4 multipliziert mit Umkehrwert der Füllvolumen), dass die Auslösekraft (bezogen auf den jeweiligen Glasbestandteil) in den bodennahen Wandbereichen größer ist als die Auslösekraft in den unverformten Wandbereichen. Bei einem Verhältnis von 1,6 zeigen die beiden Wandbereich des Vials keinen Unterschied bezüglich ihres Auslöseverhaltens (bezogen auf den jeweils bestimmten Glasbestandteil im jeweiligen Medium). Bei einem Verhältnis kleiner 1,6 ist die Auslösekraft bei einem Füllvolumen von 0,5 ml sogar kleiner als bei einem Füllvolumen von 2 ml.

Die erfindungsgemäßen Verhältnisse entsprechen somit geringen Unterschieden im Auslöseverhalten zwischen den beiden Bereichen. Somit können in den Glasfläschchen auch pharmazeutische Zubereitungen in sehr viel kleineren Dosen als es beispielsweise dem Nennvolumen des Glasfläschchens entsprechen würde aufbewahrt werden, da die durch die Migration der Glasbestandteile auftretenden Effekte auf die pharmazeutischen Wirkstoffe in den bodennahen Wandbereichen sich auf Grund der spezifischen Beschaffenheit der Glasinnenwand in diesen Bereichen von denen in unverformten Wandbereichen nur in geringem Maße unterscheiden.

Eine Weiterbildung der Erfindung sieht sogar vor, dass nach einer Lagerungszeit von 48 Wochen das Verhältnis zwischen der Konzentration des ausgelösten Bestandteiles bei einem Füllvolumen von 0,5 ml und der Konzentration bei einem Füllvolumen von 2 ml maximal 2,5, besonders bevorzugt maximal 1,5 und das Verhältnis zwischen der Konzentration bei einem Füllvolumen von 1 ml und der Konzentration bei einem Füllvolumen von 2 ml maximal 1,8, besonders bevorzugt maximal 1,5 liegt. Bevorzugt liegt das Verhältnis zwischen der Konzentration bei einem Füllvolumen von 0,5 ml und der Konzentration bei einem Füllvolumen von 2 ml im Bereich von 1 bis 1,8 und das Verhältnis zwischen der Konzentration bei einem Füllvolumen von 1 ml und der Konzentration bei einem Füllvolumen von 2 ml im Bereich von 1 bis 1,5.

In einer bevorzugten Ausführungsform der Erfindung wird als Auslösemedium zur Bestimmung der Migrationslast aufbereitetes Wasser verwendet. Unter aufbereitetem Wasser im Sinne der Erfindung wird insbesondere Wasser verstanden, dem durch lonenaustausch und/oder destillative Verfahren ein Großteil der im Wasser im nicht aufbereitetem Zustand enthaltenen Stoffe entzogen wurden. So kann es sich beispielsweise bei aufbereitetem Wasser um sog. "vollentsalzenes" Wasser (VE-Wasser) oder destilliertes Wasser handeln.

**Tabelle 1: ICP Ergebnisse für aufbereitetes Wasser als Auslösemedium**

| | B [mg/l] | Na [mg/l] | Al [mg/l] | Si [mg/l] | Ca [mg/l] |
|---|---|---|---|---|---|
| Aufbereitetes Wasser | < 0,005 | < 0,01 | < 0,005 | 0,008 ± 15% | < 0,005 |
| Bestimmungsgrenze | 0,005 | 0,01 | 0,005 | 0,005 | 0,005 |

Tabelle 1 zeigt dabei die mittels ICP-Analyse bestimmten Anteilen an Fremdstoffen des als Auslösemedium verwendeten Wassers vor dem Kontakt mit dem Glas. Im Fall von Na, B, Ca und AI liegen diese Werte unterhalb der hier möglichen Bestimmungsgrenzen.

Alternativ kann als Auslösemedium eine 15%ige KCI-Lösung verwendet werden. Hierbei wird das Glasfläschchen mit dem jeweiligen Volumen der KCI-Lösung versetzt und für einen Zeitraum von 24 Wochen bei 40°C gelagert. Bevorzugt wird hierbei als Auslösemedium eine KCI-Lösung mit den in Tabelle 2 gezeigten Konzentrationen (gemessen mittels ICP-Analyse vor Kontakt mit dem Glasvial) verwendet.

**Tabelle 2: ICP Ergebnisse für KCl-Lösung**

| Blindlösung | B [mg/l] | Na [mg/l] | Al [mg/l] | Si [mg/l] | Ca [mg/l] |
|---|---|---|---|---|---|
| 15% KCI | < 0,20 | 1,3 ± 15% | < 0,20 | < 0,30 | < 0,20 |
| Bestimmungsgrenze | 0,20 | 0,20 | 0,20 | 0,30 | 0,20 |

Nach einer Lagerungszeit von 24 Wochen und einer 15%igen KCI-Lösung als Auslösemedium beträgt das Verhältnis zwischen der Konzentration des ausgelösten Bestandteiles bei einem Füllvolumen von 0,5 ml und der Konzentration bei einem Füllvolumen von 2 ml maximal 3, besonders bevorzugt maximal 1,5 und das Verhältnis zwischen der Konzentration bei einem Füllvolumen von 1 ml und der Konzentration bei einem Füllvolumen von 2 ml maximal 1,8, besonders bevorzugt maximal 1,5.

Eine Ausführungsform dieser Alternative sieht vor, dass nach einer Lagerungszeit von 48 Wochen das Verhältnis zwischen der Konzentration des ausgelösten Bestandteiles bei einem Füllvolumen von 0,5 ml und der Konzentration bei einem Füllvolumen von 2 ml maximal 2,5, besonders bevorzugt maximal 1,5 und das Verhältnis zwischen der Konzentration bei einem Füllvolumen von 1 ml und der Konzentration bei einem Füllvolumen von 2 ml maximal 1,8, besonders bevorzugt maximal 1,5 liegt. Bevorzugt liegt das Verhältnis zwischen der Konzentration bei einem Füllvolumen von 0,5 ml und der Konzentration bei einem Füllvolumen von 2 ml im Bereich von 1 bis 2,5 und das Verhältnis zwischen der Konzentration bei einem Füllvolumen von 1 ml und der Konzentration bei einem Füllvolumen von 2 ml im Bereich von 1 bis 1,8, bevorzugt im Bereich von 1 und 1,5.

Alternativ kann als Auslösemedium eine phosphatgepufferte-Lösung mit einem pH von 7 verwendet werden, die auf der Basis von 10 mMol Natriumphosphat, 150 mMol NaCl und Tween 20 erzeugt wurde. Hierbei wird das Glasfläschchen mit dem jeweiligen Volumen der Pufferlösung versetzt und für einen Zeitraum von 24 Wochen bei 40°C gelagert.

Bevorzugt wird hierbei als Auslösemedium eine phosphatgepufferte-Lösung mit den in Tabelle 3 gezeigten Konzentrationen (gemessen mittels ICP-Analyse vor Kontakt mit dem Glasvial) verwendet.

**Tabelle 3: ICP-Konzentrationen der phosphatgepufferte-Lösung**

| Blindlösung | B [mg/l] | Al [mg/l] | Si [mg/l] | Ca [mg/l] |
|---|---|---|---|---|
| Phosphatpuffer | < 0,10 | < 0,10 | < 0,10 | < 0,10 |
| Bestimmungsgrenze | 0,10 | 0,10 | 0,10 | 0,10 |

Nach einer Lagerungszeit von 24 Wochen in phosphatgepufferter Lösung beträgt das Verhältnis zwischen der Konzentration des ausgelösten Bestandteiles bei einem Füllvolumen von 0,5 ml und der Konzentration bei einem Füllvolumen von 2 ml maximal 2,5, besonders bevorzugt maximal 2 und das Verhältnis zwischen der Konzentration bei einem Füllvolumen von 1 ml und der Konzentration bei einem Füllvolumen von 2 ml maximal 1,8, besonders bevorzugt maximal 1,6. Eine Ausführungsform dieser Alternative sieht vor, dass nach einer Lagerungszeit von 48 Wochen das Verhältnis zwischen der Konzentration des ausgelösten Bestandteiles bei einem Füllvolumen von 0,5 ml und der Konzentration bei einem Füllvolumen von 2 ml maximal 2,5, besonders bevorzugt maximal 2 und das Verhältnis zwischen der Konzentration bei einem Füllvolumen von 1 ml und der Konzentration bei einem Füllvolumen von 2 ml maximal 1,7, besonders bevorzugt maximal 1,5 beträgt. Bevorzugt liegt das Verhältnis zwischen der Konzentration bei einem Füllvolumen von 0,5 ml und der Konzentration bei einem Füllvolumen von 2 ml im Bereich von 1 bis 2,5, bevorzugt 2 bis 1 und das Verhältnis zwischen der Konzentration bei einem Füllvolumen von 1 ml und der Konzentration bei einem Füllvolumen von 2 ml im Bereich von 1 bis 1,7 liegt.

Alternativ kann als Auslösemedium eine isotonische, 0,9%ige NaCl-Lösung verwendet werden. Hierbei wird das Glasfläschchen mit dem jeweiligen Volumen der 0,9%igen NaCl-Lösung versetzt und für einen Zeitraum von 24 Wochen bei 40°C gelagert. Bevorzugt wird als Auslösemedium eine entsprechende NaCl-Lösung mit den Konzentrationen gemäß Tabelle 4 verwendet.

**Tabelle 4: ICP Ergebnisse für NaCl-Lösung**

| Blindlösung | B [mg/l] | Al [mg/l] | Si [mg/l] | Ca [mg/l] |
|---|---|---|---|---|
| 0,9% NaCl | < 0,05 | < 0,05 | < 0,05 | < 0,05 |
| Bestimmungsgrenze | 0,05 | 0,05 | 0,05 | 0,05 |

Nach einer Lagerungszeit von 24 Wochen beträgt das Verhältnis zwischen der Konzentration des ausgelösten Bestandteiles bei einem Füllvolumen von 0,5 ml und der Konzentration bei einem Füllvolumen von 2 ml maximal 2,5, besonders bevorzugt maximal 2,2 und das Verhältnis zwischen der Konzentration bei einem Füllvolumen von 1 ml und der Konzentration bei einem Füllvolumen von 2 ml maximal 1,6, besonders bevorzugt maximal 1,5. Eine Ausführungsform dieser Alternative sieht vor, dass nach einer Lagerungszeit von 48 Wochen das Verhältnis zwischen der Konzentration des ausgelösten Bestandteiles bei einem Füllvolumen von 0,5 ml und der Konzentration bei einem Füllvolumen von 2 ml maximal 2,5, besonders bevorzugt maximal 2,1 und das Verhältnis zwischen der Konzentration bei einem Füllvolumen von 1 ml und der Konzentration bei einem Füllvolumen von 2 ml maximal 1,6, besonders bevorzugt maximal 1,5 beträgt. Bevorzugt liegt das Verhältnis zwischen der Konzentration bei einem Füllvolumen von 0,5 ml und der Konzentration bei einem Füllvolumen von 2 ml im Bereich von 1 bis 2,5, bevorzugt im Bereich von 1 bis 2,2 und das Verhältnis zwischen der Konzentration bei einem Füllvolumen von 1 ml und der Konzentration bei einem Füllvolumen von 2 ml im Bereich von 1 bis 1,5.

Alternativ kann als Auslösemedium eine 8,4%ige Natriumbicarbonat-Lösung verwendet werden. Hierbei wird das Glasfläschchen mit dem jeweiligen Volumen der Natriumbicarbonat-Lösung versetzt und für einen Zeitraum von 24 Wochen bei 40°C gelagert. Bevorzugt wird in dieser Ausführungsform als Auslösemedium eine Natriumbicarbonat-Lösung mit den in Tabelle 5 gezeigten Konzentrationen verwendet.

**Tabelle 5: ICP-Konzentrationen Natriumbicarbonat-Lösung**

| Blindlösung | B [mg/l] | Al [mg/l] | Si [mg/l] | Ca [mg/l] |
|---|---|---|---|---|
| 8,4% NaHCO₃ | < 0,10 | < 0,10 | 1,4 ± 10% | 4,2 ± 10% |
| Bestimmungsgrenze | 0,10 | 0,10 | 0,50 | 1,25 |

Nach einer Lagerungszeit von 24 Wochen beträgt hier das Verhältnis zwischen der Konzentration des ausgelösten Bestandteiles bei einem Füllvolumen von 0,5 ml und der Konzentration bei einem Füllvolumen von 2 ml maximal 4,5 besonders bevorzugt maximal 1,5 und das Verhältnis zwischen der Konzentration bei einem Füllvolumen von 1 ml und der Konzentration bei einem Füllvolumen von 2 ml maximal 2,1, besonders bevorzugt maximal 1,4. Bevorzugt liegt das Verhältnis zwischen der Konzentration bei einem Füllvolumen von 0,5 ml und der Konzentration bei einem Füllvolumen von 2 ml im Bereich von 1 bis 4,5 oder sogar im Bereich von 1 und 1,5 und das Verhältnis zwischen der Konzentration bei einem Füllvolumen von 1 ml und der Konzentration bei einem Füllvolumen von 2 ml im Bereich von 1 bis 2,1, bevorzugt im Bereich von 1 und 1,4.

Gemäß einer Ausführungsform der Erfindung beträgt das Verhältnis zwischen der Konzentration des ausgelösten Bestandteils bei einem Füllvolumen von 0,5 ml und der Konzentration bei einem Füllvolumen von 1 ml nach einer Auslaugzeitraum von 24 Wochen mit destilliertem Wasser bei 40°C maximal 2,5, bevorzugt maximal 1,7. insbesondere liegt das Verhältnis zwischen der Konzentration des ausgelösten Bestandteils bei einem Füllvolumen von 0,5 ml und der Konzentration bei einem Füllvolumen von 1 ml im Bereich von 1 und 2,5 oder sogar 1 und 1,7.

Eine Weiterbildung der Erfindung sieht vor, dass das Glasfläschchen aus einem Mehrkomponentenglas umfassend zumindest eines der Bestandteile aus der Gruppe gebildet von Si, B, Al, Na, K und/oder Ca, besteht. Gemäß einer Ausführungsform dieser Weiterbildung handelt es sich bei dem ausgelösten Glasbestandteil um zumindest einen der Bestandteile aus der Gruppe mit den Elementen Si, B, Al, Na, K und Ca.

Insbesondere ist das Mehrkomponentenglas ein Borosilikatglas, bevorzugt ein Neutralglas-Glas. Als besonders vorteilhaft hat sich die Verwendung von Neutralgläsern mit einer Hydrolysebeständigkeit der Klasse I herausgestellt. Unter Neutralglas wird ein Borosilikatglas verstanden, welches signifikante Anteile an B₂O₃, Al₂O₃, Alkalimetalloxide und/oder Erdalkalioxide aufweist. Auf Grund ihrer chemischen Zusammensetzung weisen Neutralgläser dabei eine hohe Hydrolysebeständigkeit auf. Unter der Hydrolsysebeständigkeit wird dabei insbesondere die Beständigkeit gegen ein Auslösen von löslichen Glasbestandteilen, insbesondere von Ionen, verstanden. Die Hydrolysebeständigkeit des Glases kann beispielsweise durch Titration der entsprechenden ausgelösten Bestandteile im Auslösemedium, d.h. in der Flüssigkeit, die unter den jeweiligen Testbedingungen in Kontakt mit der Glasoberfläche gekommen ist, quantifiziert werden. Eine Bestimmung kann hierbei an einer Glasoberfläche eines entsprechenden Glasfläschchens bzw. Vials oder aber auch an Glaskörnern (ISO 719 oder ISO720) bestimmt werden.

Als vorteilhaft hat sich die Verwendung eines Glases mit den folgenden Bestandteilen in Gewichts-% herausgestellt:

| | |
|---|---|
| B₂O₃ | > 8, bevorzugt 8 - 12 |
| SiO₂ | 65 - 85, bevorzugt 70 - 80 |
| Na₂O + K₂O | 4 - 8 |
| MgO + CaO + BaO + SrO | 0 - 5 |
| Al₂O₃ | 2 - 7 |

In einer besonders bevorzugten Ausführungsform der Erfindung weist das Glas eine Zusammensetzung mit den folgenden Bestandteilen in Gewichts-% auf:

| | |
|---|---|
| B₂O₃ | 10,5 |
| SiO₂ | 75 |
| Na₂O + K₂O | 7 |
| MgO + CaO + BaO + SrO | 1,5 |
| Al₂O₃ | 5 |

Gemäß einer Ausführungsform beträgt bei Auslösen des Glasfläschchens mit aufbereitetem Wasser bei einer Temperatur von 40°C und einer Lagerzeit von 24 Wochen das Verhältnis zwischen der Konzentration des ausgelösten Bestandteils bei einem Füllvolumen von 0,5 ml und der Konzentration des ausgelösten Bestandteils bei einem Füllvolumen von 2 ml für Silizium maximal 1,5, für Natrium maximal 2,5 und/oder für Bor maximal 3. Bevorzugt weist das als Auslösemedium verwendete, aufbereitete Wasser die in Tabelle 1 aufgeführten Konzentrationen auf.

Insbesondere liegt das Verhältnis zwischen der Konzentration des ausgelösten Bestandteils bei einem Füllvolumen von 1 ml und der Konzentration des ausgelösten Bestandteils bei einem Füllvolumen von 2 ml für Silizium im Bereich von 1 bis 1,5, für Natrium im Bereich von 1 bis 2,1 und/oder für Bor im Bereich von 1 bis 2,5.

Alternativ oder zusätzlich beträgt bei Auslösen des Glasfläschchens mit aufbereitetem Wasser bei einer Temperatur von 40°C und einer Lagerzeit von 24 Wochen das Verhältnis zwischen der Konzentration des ausgelösten Bestandteils bei einem Füllvolumen von 0,5 ml und der Konzentration des ausgelösten Bestandteils bei einem Füllvolumen von 1 ml für Silizium maximal 1,5, für Natrium maximal 1,6 und/oder für Bor maximal 2.

Gemäß einer Ausführungsform beträgt bei Auslösen des Glasfläschchens mit aufbereitetem Wasser bei einer Temperatur von 40°C und einer Lagerzeit von 24 Wochen die Konzentration des ausgelösten Bestandteils bei einem Füllvolumen von 0,5 ml für Silizium maximal 6 mg/l, für Natrium maximal 3 mg/l, für Aluminium maximal 0,6 mg/l, für Calcium maximal 0,2 mg/l und/oder für Bor maximal 1,3 mg/l. Bevorzugt weist das als Auslösemedium verwendete, aufbereitete Wasser die in Tabelle 1 aufgeführten Konzentrationen auf. Eine weitere Ausführungsform sieht vor, dass bei Auslösen des Glasfläschchens mit aufbereitetem Wasser bei einer Temperatur von 40°C und einer Lagerzeit von 24 Wochen bei einem Füllvolumen von 0,5 ml die Konzentration des ausgelösten Bestandteiles für Silizium im Bereich von 3 bis 6 mg/l, für Bor im Bereich von 0,8 bis 1,6 mg/l, für Natrium im Bereich von 1,6 bis 4 mg/l, für Calcium im Bereich von 0,05 bis 0,5 mg/l und/oder für Aluminium im Bereich von 0,1 bis 1 mg/l liegt.

Alternativ oder zusätzlich liegen nach einem Auslösen des Glasfläschchens unter den oben genannten Bedingungen und einem Füllvolumen von 1 ml die Konzentration des ausgelösten Bestandteiles für Silizium im Bereich von 3 bis 6 mg/l, für Bor im Bereich von 0,4 bis 1 mg/l, für Natrium im Bereich von 1,5 bis 2,5 mg/l für Calcium im Bereich von 0,05 bis 0,25 mg/l und/oder für Aluminium im Bereich von 0,1 bis 0,7 mg/ml.

Durch diese geringen Konzentrationen kann auch bei längerer Lagerung der pharmazeutischen Zubereitung eine Beeinflussung der Wirksamkeit durch migrierende Glasbestandteile vermieden werden.

Gemäß einer weiteren Ausführungsform beträgt bei Auslösen des Glasfläschchens mit einer 15%igen KCI-Lösung, insbesondere mit einer KCI-Lösung gemäß Tabelle 2, bei einer Temperatur von 40°C und einer Lagerzeit von 24 Wochen mit einem Füllvolumen von 0,5 ml die Konzentration des ausgelösten Bestandteils bei einem Füllvolumen von 0,5 ml für Silizium maximal 3 mg/l, für Natrium maximal 3,5 mg/l, für Calcium maximal 0,6 mg/l und/oder für Bor maximal 1,3 mg/l. Bevorzugt liegt die Konzentration des ausgelösten Bestandteiles für Silizium im Bereich von 1 bis 3 mg/l, für Bor im Bereich von 0,2 bis 1,2 mg/l, für Natrium im Bereich von 1,8 bis 3,5 mg/l und/oder für Calcium im Bereich von 0,2 bis 1 mg/l.

Alternativ oder zusätzlich beträgt bei Auslösen des Glasfläschchens mit einer 15%igen KCI-Lösung, insbesondere mit einer KCI-Lösung gemäß Tabelle 2, bei einer Temperatur von 40°C und einer Lagerzeit von 24 Wochen mit einem Füllvolumen von 1 ml die Konzentration des ausgelösten Bestandteils für Silizium maximal 2 mg/l, für Natrium maximal 3 mg/l, für Calcium maximal 0,5 mg/l und/oder für Bor maximal 1,0 mg/l. Bevorzugt liegt die Konzentration des ausgelösten Bestandteiles für Silizium im Bereich von 1 bis 2 mg/l, für Bor im Bereich von 0,2 bis 1,0 mg/l, für Natrium im Bereich von 1,8 bis 3 mg/l und/oder für Calcium im Bereich von 0,2 bis 0,5 mg/l.

Eine weitere Ausführungsform sieht vor, dass bei Auslösen des Glasfläschchens mit einer 0,9%igen NaCl-Lösung, insbesondere mit einer NaCl-Lösung gemäß Tabelle 3, bei einer Temperatur von 40°C und einer Lagerzeit von 24 Wochen mit einem Füllvolumen von 0,5 ml die Konzentration des ausgelösten Bestandteils bei einem Füllvolumen von 0,5 ml für Silizium maximal 4 mg/l, für Calcium maximal 0,6 mg/l und/oder für Bor maximal 1,3 mg/l beträgt. Bevorzugt liegt die Konzentration des ausgelösten Bestandteiles für Silizium im Bereich von 2 bis 4 mg/l, für Bor im Bereich von 0,6 bis 1,5 mg/l, und/oder für Calcium im Bereich von 0,2 bis 1 mg/l.

Alternativ oder zusätzlich beträgt bei Auslösen des Glasfläschchens mit 0,9%igen NaCl-Lösung, insbesondere mit einer NaCl-Lösung gemäß Tabelle 3, bei einer Temperatur von 40°C und einer Lagerzeit von 24 Wochen mit einem Füllvolumen von 1 ml die Konzentration des ausgelösten Bestandteils für Silizium maximal 3,5 mg/l, für Calcium maximal 0,5 mg/l und/oder für Bor maximal 1,5 mg/l beträgt. Bevorzugt liegt die Konzentration des ausgelösten Bestandteiles für Silizium im Bereich von 2 bis 3,5 mg/l, für Bor im Bereich von 0,2 bis 1,3 mg/l, und/oder für Calcium im Bereich von 0,2 bis 0,5 mg/l.

Gemäß einer weiteren Ausführungsform beträgt bei Auslösen des Glasfläschchens mit einer 8,4%igen NaHCOs-Lösung, insbesondere mit einer 8,5%igen NaHCOs-Lösung gemäß Tabelle 4, bei einer Temperatur von 40°C und einer Lagerzeit von 24 Wochen mit einem Füllvolumen von 0,5 ml die Konzentration des ausgelösten Bestandteils bei einem Füllvolumen von 0,5 ml für Silizium maximal 15 mg/l, für Calcium maximal 2,8 mg/l und/oder für Bor maximal 3 mg/l. Bevorzugt liegt die Konzentration des ausgelösten Bestandteiles für Silizium im Bereich von 3 bis 15 mg/l und/oder für Bor im Bereich von 0,2 bis 3 mg/l.

Alternativ oder zusätzlich beträgt bei Auslösen des Glasfläschchens mit der oben genannten NaHCO₃-Lösung, bei einer Temperatur von 40°C und einer Lagerzeit von 24 Wochen mit einem Füllvolumen von 1 ml die Konzentration des ausgelösten Bestandteils für Silizium maximal 7 mg/l, für Calcium maximal 5 mg/l und/oder für Bor maximal 1,5 mg/l. Bevorzugt liegt die Konzentration des ausgelösten Bestandteiles für Silizium im Bereich von 3 bis 10 mg/l, und/oder für Bor im Bereich von 0,2 bis 1,5 mg/l.

Eine weitere Ausführungsform sieht vor, dass bei Auslösen des Glasfläschchens mit einer phosphatgepufferten Lösung mit einem pH Wert von 7 als Auslösemedium, insbesondere mit einer entsprechenden Pufferlösung gemäß Tabelle 5, bei einer Temperatur von 40°C und einer Lagerzeit von 24 Wochen mit einem Füllvolumen von 0,5 ml die Konzentration des ausgelösten Bestandteils bei einem Füllvolumen von 0,5 ml für Silizium maximal 10 mg/l, für Calcium maximal 1 mg/l und/oder für Bor maximal 3 mg/l beträgt. Bevorzugt liegt die Konzentration des ausgelösten Bestandteiles für Silizium im Bereich von 5 bis 10 mg/l und/oder für Bor im Bereich von 0,5 bis 2,5 mg/l.

Alternativ oder zusätzlich beträgt bei Auslösen des Glasfläschchens mit der oben genannten NaHCOs-Lösung, bei einer Temperatur von 40°C und einer Lagerzeit von 24 Wochen mit einem Füllvolumen von 1 ml die Konzentration des ausgelösten Bestandteils für Silizium maximal 8 mg/l, für Calcium maximal 0,5 mg/l und/oder für Bor maximal 2 mg/l. Bevorzugt liegt die Konzentration des ausgelösten Bestandteiles für Silizium im Bereich von 8 bis 4 mg/l, und/oder für Bor im Bereich von 3 bis 0,2 mg/l.

Durch diese geringen Konzentrationen in unterschiedlichen Flüssigkeiten als Auslösemedien kann auch bei längerer Lagerung der pharmazeutischen Zubereitung eine Beeinflussung der Wirksamkeit durch migrierende Glasbestandteile verhindert werden. Durch die geringe Migrationslast auch in bodennahen Bereichen eigenen sich die Glasfläschchen insbesondere zur Verwendung zur Befüllung mit pharmazeutischen Zubereitungen mit geringen Befüllungsgraden. So sieht eine Ausführungsform der Erfindung vor, dass der Befüllungsgrad maximal 0,125 oder sogar nur maximal 0,1 bezogen auf das Randvollvolumen beträgt.

Das Glasfläschchen weist ein Randvollvolumen kleiner 4,5 ml auf. Bevorzugt liegt das entsprechende Nennvolumen im Bereich von 1 bis 2 ml. Bei einem Glasfläschchen mit einem Nennvolumen von 2 ml liegt der Nennbefüllungsgrad, d.h. das Verhältnis von Füllvolumen zu Nennvolumen bevorzugt bei maximal 0,5 oder sogar maximal 0,25.

Eine Weiterbildung der Erfindung sieht vor, dass das Glasfläschchen (1) aus einem borhaltigem Mehrkomponentenglas besteht und die mittlere Konzentration der Borionen, gemessen anhand eines Konzentrationstiefenprofils bei einer Tiefe im Bereich von 10 bis 30 nm für Borionen einen Mittelwert aufweist, welcher eine Überhöhung von maximal 30 % , bevorzugt maximal 25% und besonders bevorzugt maximal 20% gegenüber einer mittleren Konzentration von Borionen gemessen anhand eines Konzentrationstiefenprofils bei einer Tiefe im Bereich von 10 bis 30 nm in der Behältermitte aufweist, wobei die Behältermitte von der Unterseite des Bodens in Richtung auf die Öffnung des Fläschchens bestimmt wird. Gemäß einer Ausführungsform liegt die Überhöhung im Konzentrationsprofil der Borionen in Bereich von 10 bis 25%.

Insbesondere kann das Konzentrationstiefenprofil bei einer Tiefe im Bereich von 10 bis 30 nm in einem umgeformten bodennahen Wandbereich für Borionen eine Überhöhung von maximal 30 % gegenüber einem oberen, nicht umgeformten Wandbereich aufweisen. Bevorzugt weist das Konzentrationstiefenprofil bei einer Tiefe von 10 bis 30 nm in einem umgeformten bodennahen Wandbereich für Borionen eine Überhöhung von maximal 25 % oder sogar nur 20 % gegenüber einem oberen Wandbereich auf. Unter dem bodennahen Wandbereich wird im Sinne der Erfindung der Bereich der Innenwand des Glasfläschchens verstanden, welcher von der Außenseite, beziehungsweise Unterseite des Glasbodens 1 bis 5 mm, bevorzugt 1 bis 3 mm beabstandet ist. Der obere, nicht umgeformte Wandbereich ist dabei insbesondere 8 bis 20 mm, bevorzugt 10 bis 15 mm vom Boden des Glasfläschchens beabstandet.

Das Konzentrationstiefenprofil wurde dabei mittels TOF-Sekundärionen-Massenspektroskopie (TOF-SIMS) im Rahmen der ISO 17025 und spezifisch gemäß der ISO 18116 ermittelt. Zur Tiefenkalibrierung wurde die Analysentiefe über die Sputterzeit aus der Abtragsrate bestimmt. Diese Abtragsrate wurde an einem Referenzglas bestimmt. Die äußeren 5 nm des Glases wurden für die Auswertung nicht berücksichtigt, da hier Oberflächenverunreinigungen sowie noch nicht vollständig ausgebildeter Ladungs-Sputtergleichgewichte vorliegen können.

Unter dem bodennahen Wandbereich wird im Sinne der Erfindung der Bereich der Innenwand des Glasfläschchens verstanden, welcher von der Außenseite, beziehungsweise Unterseite des Glasbodens 1 bis 5 mm, bevorzugt 1 bis 3 mm beabstandet ist. Der obere, nicht umgeformte Wandbereich ist dabei insbesondere 8 bis 20 mm, bevorzugt 10 bis 15 mm vom Boden des Glasfläschchens beabstandet.

Eine Überhöhung der Borkonzentration in den bodennahen Bereichen ist dabei insbesondere darauf zurück zu führen, dass beim Umformprozess zur Herstellung der Glasfläschchen auf Grund der hohen Temperaturen Glasbestandteile, insbesondere Borate, aus dem Boden ausdampfen und anschließend auf Grund des Temperaturgradienten zwischen Boden und bodennahen Wandbereichen in die bodennahen Wandbereiche eindiffundieren und so zu einer Erhöhung der Borionenkonzentration in den oberflächennahen Glasschichten führen.

Dies kann sich insofern nachteilig auf die chemische Stabilität und das Auslöseverhalten des Glases im bodennahen Wandbereich auswirken, da es durch die erhöhte Borkonzentration in den oberflächennahen Glasschichten zu einer Mischungslücke im Phasendiagramm kommen kann. Dies kann bei der Abkühlung der oberflächennahen Glasschicht eine Phasentrennung zur Folge haben. Neben einer geringeren mechanischen Stabilität kann eine Phasentrennung auch zu einer verstärkten Migration von Glasbestandteilen in das Füllmedium führen. Dies kann beispielsweise durch eine schwächere Einbindung von einzelnen Glasbestandteilen in die jeweilige Phase erfolgen, was zu einer erhöhten Mobilität der jeweiligen Bestandteile führen kann.

Eine Ausführungsform der Erfindung sieht vor, dass das Glasfläschchen im bodennahen Wandbereiche ab einer Tiefe von 150 nm, bevorzugt ab einer Tiefe von 100 nm und besonders bevorzugt ab einer Tiefe von 50 nm für die Konzentration der Borionen einen Plateauwert aufweist. Unter einem Plateauwert werden insbesondere weitgehend konstante Werte verstanden, die nicht mehr als 20%, bevorzugt nicht mehr als 10% vom Mittelwert des konstanten Wertes für größere Tiefen (> 200 nm) abweichen.

Durch die erfindungsgemäße geringe Überhöhung der Borionenkonzentration sowie den Konzentrationsverlauf in der Glaswandung wird eine Phasentrennung vermieden, so dass das Glas auch im bodennahen Wandbereich eine hohe chemische und mechanische Stabilität aufweist. Gemäß einer Ausführungsform ist das Glas der gesamten Innenwandung des Glasfläschchens, also auch in einem bodennahen Wandbereich, bis zu einer Tiefe von zumindest 200 nm einphasig.

Die beschriebenen Glasfläschchen können beispielsweise mit Hilfe des folgenden Herstellungsverfahrens erhalten werden. Das Herstellungsverfahren umfasst hierbei zumindest die folgenden Schritte
- lokales Erwärmen eines Endes eines Glasrohrs,
- Abtrennen des lokal erwärmten Endes des Glasrohrs unter Ausbildung eines Glasfläschchens mit einem geschlossenen Boden, und
- weitere Formung des Bodens des Glasfläschchens.

Hierbei wird das ausgebildete Glasfläschchen nach dem Abtrennen von dem Glasrohr bevorzugt kopfüber gehalten und bei der weiteren Formung des Bodens wird mit Hilfe eines Spülgases eine Spülgasströmung im Inneren des Glasfläschchens erzeugt. Dadurch kommt es nicht zu einer Diffusion der Borate in die Glasoberfläche der bodennahen Bereiche.

Gemäß einer Ausführungsform strömt das Spülgas über die Einfüllöffnung mittig ein- oder aus und außermittig aus oder ein, so dass sich ein Staudruck ausbildet. Durch dieses Strömungsprofil werden Borate, welche während des Umformprozesses aus dem Boden ausdampfen, besonders gut mit dem Spülgas aus dem Glasfläschchen geleitet.

Durch die hohe chemische Stabilität der Glasinnenwand auch im bodennahen Bereich kann auf weitere Maßnahmen wie beispielsweise eine Ammoniumsulfatbehandlung oder einen Ätzprozess verzichtet werden. Ebenso weist die Oberfläche der Innenwand gemäß einer Ausführungsform keine Beschichtung auf.

Das Glasfläschchen zeigt durch sein Auslöseverhalten, insbesondere auf Grund der spezifischen Eigenschaften der Glasoberfläche in den bodennahen Randbereichen, nur geringe Wechselwirkungen mit pharmazeutischen Wirkstoffen wie beispielsweise therapeutische Proteine, monoklonale Antikörper oder Vakzine. Gemäß einer Ausführungsform enthält die pharmazeutische Wirkstoffzubereitung, mit der das Glasfläschchen befüllt ist daher therapeutische Proteine, monoklonale Antikörper und/oder Vakzine.

Gemäß einer Ausführungsform der Erfindung weist das Glas am Boden an der Innenwandung eine Zusammensetzung mit einem höheren SiO₂-Anteil als an der Seitenwand und an deren Übergang in den Boden aufweist.

Gemäß einer Ausführungsform der Erfindung ist die Konzentration der Siliziumionen gemessen an einem Meßort an der Bodeninnenseite des Glasfläschchens um zumindest 10%, bevorzugt um zumindest 15% gegenüber einem Meßort in der Ebene der Behältermitte oder einem oberen Wandbereich erhöht. Zur Ermittlung der Konzentrationsüberhöhung wird hierbei ein Konzentrationstiefenprofil bei einer Tiefe im Bereich von 5 bis 15 nm erstellt. Aus den so erhaltenen Meßdaten wird der Mittelwert erhalten und mit dem entsprechenden Mittelwert aus einem Meßort in der Behältermitte verglichen. Die Lage der Ebene der Behältermitte wird dabei von der Unterseite des Bodens in Richtung auf die Öffnung des Fläschchens bestimmt. Insbesondere weist ein SIMS-Konzentrationstiefenprofil des Glases im Bereich des Bodens bei einer Tiefe im Bereich von 5 bis 15 nm für Siliziumionen eine Überhöhung von zumindest 10 %, bevorzugt von zumindest 20 % gegenüber einem oberen Wandbereich auf.

Die Konzentration von SiO₂ im Boden des Glasfläschchens kann hierbei zumindest um den Faktor 1,2 oder sogar zumindest um den Faktor 1,3 gegenüber der SiO₂-Konzentration in einem oberen Wandbereich des Glasfläschchens erhöht sein. Bevorzugt liegt der Faktor im Bereich von 1,1 und 1,4.

Durch den hohen Siliziumanteil weist der Boden des Glasfläschchens eine hohe chemische Stabilität auf. Die Erhöhung des Siliziumanteils korreliert zudem mit einer Verarmung des Glases an anderen Glasbestandteilen. Im Fall von Borosilikaten handelt es sich hierbei insbesondere um Borionen und Alkaliionen, welche während des Umformprozesses zur Herstellung des Bodens ausdampfen.

Gemäß einer Ausführungsform ist die über die Meßwerte gemittelte Konzentration der Natriumionen gemessen an einem Meßort an der Bodeninnenseite anhand eines Konzentrationstiefenprofils bei einer Tiefe im Bereich von 5 bis 15 nm um zumindest den Faktor 1,5 bevorzugt um zumindest den Faktor 2, besonders bevorzugt um zumindest den Faktor 2,5 kleiner als der entsprechend ermittelte Mittelwert der Natriumkonzentration an einem Meßort in der Ebene der Behältermitte. Bevorzugt liegt der Faktor im Bereich von 1,6 und 2,2. Eine Ausführungsform sieht vor, dass das SIMS-Tiefenprofil des Glases im Bereich des Bodens bei einer Tiefe im Bereich von 5 bis 15 nm für Natriumionen eine Verringerung von zumindest 20%, bevorzugt zumindest 40% gegenüber einem oberen Wandbereich aufweist. Die Konzentration von Natrium im Boden des Glasfläschchens kann hierbei insbesondere zumindest um den Faktor 1,5 oder sogar zumindest um den Faktor 1,8 und besonders bevorzugt sogar zumindest um den Faktor 2,5 gegenüber der Natriumkonzentration in einem oberen Wandbereich des Glasfläschchens verringert sein.

Des Weiteren kann der Boden des Glasfläschchens gegenüber einem oberen Wandbereich des Glasfläschchens eine verringerte Calciumkonzentration aufweisen. Insbesondere kann der Mittelwert der Konzentration, ermittelt durch ein SIMS-Konzentrationstiefenprofil für Calcium bei einer Tiefe im Bereich von 10 bis 30 nm, am Boden des Glasfläschchens eine Verringerung von zumindest 20%, bevorzugt zumindest 30% gegenüber dem entsprechend ermittelten Mittelwert der Konzentration in einem oberen Wandbereich des Glasfläschchens aufweisen. Insbesondere kann die Konzentration von Calcium im Boden des Glasfläschchens zumindest um den Faktor 1,3 oder sogar zumindest um den Faktor 1,6 gegenüber der Calciumkonzentration in einem oberen Wandbereich des Glasfläschchens verringert sein.

Alternativ oder zusätzlich kann der Boden des Glasfläschchens gegenüber einem oberen Wandbereich des Glasfläschchens eine verringerte Borkonzentration aufweisen. Insbesondere kann das SIMS-Tiefenprofil für Bor bei einer Tiefe im Bereich von 10 bis 30 nm für Borionen eine Verringerung von zumindest 60%, bevorzugt zumindest 80% aufweisen. So kann die Konzentration der Borionen, gemessen an einem Meßort an der Bodeninnenseite anhand eines Konzentrationstiefenprofils bei einer Tiefe im Bereich von 10 bis 30 nm einen über die Meßwerte des Konzentrationstiefenprofils gemittelten Wert aufweisen, welcher eine Verringerung zum zumindest den Faktor 3 bevorzugt um zumindest den Faktor 2, besonders bevorzugt um zumindest den Faktor 5 gegenüber einer Konzentration von Borionen gemessen anhand eines Konzentrationstiefenprofils bei einer Tiefe im Bereich von 10 bis 30 nm mit einem Meßort in der Ebene der Behältermitte aufweist, wobei die Lage der Ebene der Behältermitte von der Unterseite des Bodens in Richtung auf die Öffnung des Fläschchens bestimmt wird.

Das Glasfläschchen weist gemäß diesen Ausführungsformen somit eine inhomogene Konzentrationsverteilung von Glasbestandteilen bezogen auf die unterschiedlichen Bereiche Boden, bodennaher Wandbereich und oberer Wandbereich auf. Dies ist vorteilhaft, da der Boden eine verringerte Konzentration an auslösbaren Glasbestandteilen wie Bor, Alkali- oder Erdalkaliionen aufweist. Somit ist auch die Migrationslast, die vom Boden des Glasfläschchens ausgeht, geringer als die Migrationslast der übrigen Bereiche des Glasfläschchens. Bei geringen Füllgraden und in Zusammenhang mit der üblichen stehenden Lagerung der Pharmapackmittel wirkt sich dieser positive Effekt besonders stark aus, da hier stets der Boden von der Flüssigkeit bedeckt wird während nur ein geringer Anteil der Wandoberfläche in Kontakt mit der Flüssigkeit kommt.

Bevorzugt weist das Glasfläschchen mit der pharmazeutischen Zubereitung einen Verschluss, bevorzugt einen Sterilverschluss auf.

Des Weiteren betrifft die Erfindung ein Medizinprodukt, umfassend ein entsprechendes mit einer flüssigen pharmazeutischen Wirkstoffzubereitung befülltes und verschlossenes Glasfläschchen.

### Detaillierte Beschreibung der Erfindung

Nachfolgend wird die Erfindung an Hand von Figuren und Ausführungsbeispielen näher beschrieben. Es zeigen:
Fig. 1 eine schematische Darstellung eines Glasfläschchens,
Fig. 2 bis Fig. 5 das Auslösungsverhalten eines Glasfläschchens als Ausführungsbeispiel sowie eines aus dem Stand der Technik bekannten Glasfläschchens für Borionen bei unterschiedlichen Flüssigkeiten als Auslösemedium,
Fig. 6 bis Fig. 10 das Auslösungsverhalten bezüglich Siliziumionen bei unterschiedlichen Flüssigkeiten als Auslösemedium,
Fig. 11 bis Fig. 12 das Auslösungsverhalten bezüglich Natrium bei unterschiedlichen Flüssigkeiten als Auslösemedium,
Fig. 13 bis Fig. 16 das Auslösungsverhalten bezüglich Calcium bei unterschiedlichen Flüssigkeiten als Auslösemedium,
Fig. 17 bis Fig. 21 die ausgelöste Borionenkonzentration eines Ausführungsbeispiels sowie eines aus dem Stand der Technik bekannten Glasfläschchens bei unterschiedlichen Flüssigkeiten als Auslösemedium,
Fig. 22 bis Fig. 26 die ausgelöste Siliziumkonzentration eines Ausführungsbeispiels sowie eines aus dem Stand der Technik bekannten Glasfläschchens bei unterschiedlichen Flüssigkeiten als Auslösemedium,
Fig. 27 bis Fig. 28 die ausgelöste Natriumionenkonzentration eines Ausführungsbeispiels sowie eines aus dem Stand der Technik bekannten Glasfläschchens bei unterschiedlichen Flüssigkeiten als Auslösemedium,
Fig. 29 bis Fig. 30 die ausgelöste Calciumionenkonzentration eines Ausführungsbeispiels sowie eines aus dem Stand der Technik bekannten Glasfläschchens bei unterschiedlichen Flüssigkeiten als Auslösemedium
Fig. 31 die ausgelöste Aluminiumionenkonzentration eines Ausführungsbeispiels sowie eines aus dem Stand der Technik bekannten Glasfläschchens bei Auslösung mit Wasser
Fig. 32 SIMS-Intensitäts-Tiefenprofile unterschiedlicher Bereiche eines aus dem Stand der Technik bekannten Glasfläschchens,
Fig. 33 SIMS-Konzentrationsprofile für Borionen aus dem bodennahen Wandbereich eines Glasfläschchens zur erfindungsgemäßen Verwendung sowie eines aus dem Stand der Technik bekannten Glasfläschchens,
Fig. 34a bis 34d eine schematische Darstellung von vier Phasen des Ausblasprozesses eines Verfahrens zur Herstellung der erfindungsgemäßen Glasfläschchen
Fig. 35 eine SEM-Querschnitts-Aufnahme des bodennahen Bereiches eines Glasfläschchens zur erfindungsgemäßen Verwendung
Fig. 36 eine SEM-Querschnitts-Aufnahme des bodennahen Bereiches eines aus dem Stand der Technik bekannten Glasfläschchens und.
Fig. 37 und Fig. 38 SIMS-Intensitäts-Tiefenprofile für Bor und Natrium aus dem oberen Wandbereich (5) und dem Boden (7) eines Glasfläschchens

### Bezugszeichenliste

- 1: Glasfläschchen
- 2: Flaschenhals
- 3: Boden
- 4: Flüssigkeit
- 5: oberer Wandbereich
- 6: unterer Wandbereich
- 7: Innenwandung des Bodens
- 9: Füllvolumen
- 10: Flaschenhals
- 11: Randvollvolumen
- 12: Mittenebene
- 13: Bodenunterseite
- 14: herkömmliches Vial
- 15: Vial gemäß einer Ausführungsform
- 16: Reaktionszone
- 20: Innenwandung
- 21: Außenwandung
- 50, 51, 52, 52: Spülgasströmung
- 54: heiße Zone
- 60, 61: Konzentrationstiefenprofil Vergleichsvial
- 70, 71: Konzentrationstiefenprofil Ausführungsbeispiel
- 200: Rohr

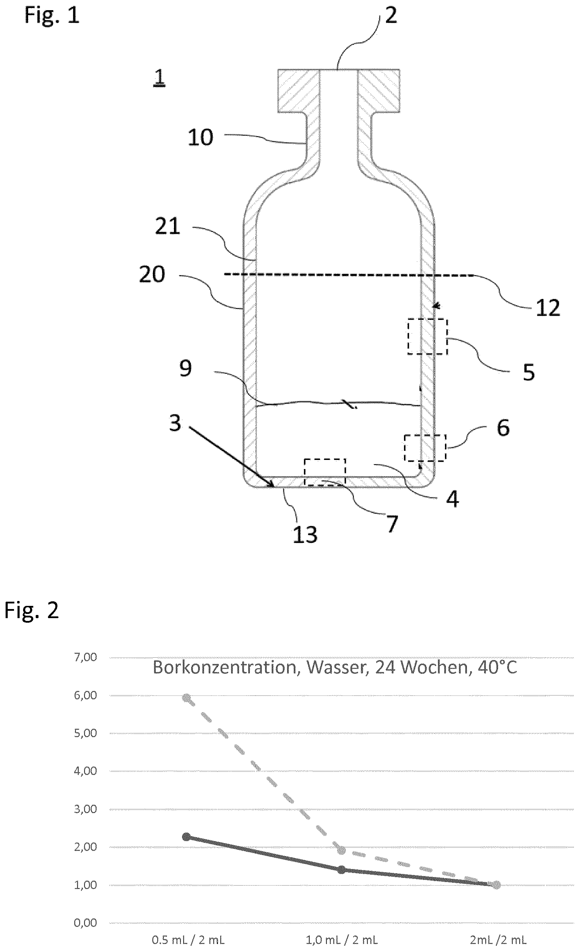

In Fig. 1 ist ein schematischer Querschnitt eines Glasfläschchens 1 dargestellt, welches mit einer Flüssigkeit 4 befüllt ist. Das Glasfläschchen 1 umfasst einem Boden 3 und eine Wandung 20, 21, welche im oberen Bereich des Glasfläschchens 1 in einen Halsbereich 10 übergeht und mit dem Rand 11 abschließt. Die Wandung bildet eine Außenwandung 20 und eine Innenwandung 20, wobei nur die Innenwandung 20 mit der Flüssigkeit 4 in Kontakt kommt. Die Ebene der Behältermitte 12 wird von der Unterseite des Bodens 13 bestimmt.

Das Glasfläschchen 1 weist ein Randvollvolumen < 4,5 ml auf, wobei unter dem Randvollvolumen das gesamte Innenvolumen des Glasfläschchens bis zum oberen Rand 11 verstanden wird. Das tatsächliche Füllvolumen 9 wird durch das Volumen der Flüssigkeit 4 bestimmt. Erfindungsgemäß ist das Füllvolumen 9 zumindest um den Faktor 4 kleiner als das Randvollvolumen 11. Der Befüllungsgrad des Glasfläschchens 1 als Quotient zwischen Füllvolumen 9 und Randvollvolumen 11 ist daher kleiner 0,25.

Der geringe Befüllungsgrad hat zur Folge, dass die Flüssigkeit vorwiegend die Innenwandung 7 des Bodens sowie den bodennahen Wandbereich 6 bedeckt. Die Innenwandung des Bodens 7 und der bodennahe Wandbereich sind dabei die Bereiche des Glasfläschchens, welche beim Umformungsprozess zur Herstellung des Fläschchens auf Grund der hohen Prozesstemperaturen in ihrer Zusammensetzung am stärksten beeinflusst werden. Im Gegensatz dazu werden Wandbereiche, wie beispielsweise der obere Wandbereich 5, die weiter vom Boden 7 beabstandet sind, durch den Herstellungsprozess weniger stark beeinflusst.

Der bodennahe Wandbereich 6 weist einen Abstand im Bereich von 0,5 bis 5 mm und der obere Wandbereich 5 einen Abstand im Bereich von 10 bis 20 mm von der Bodenaußenwand 7 auf.

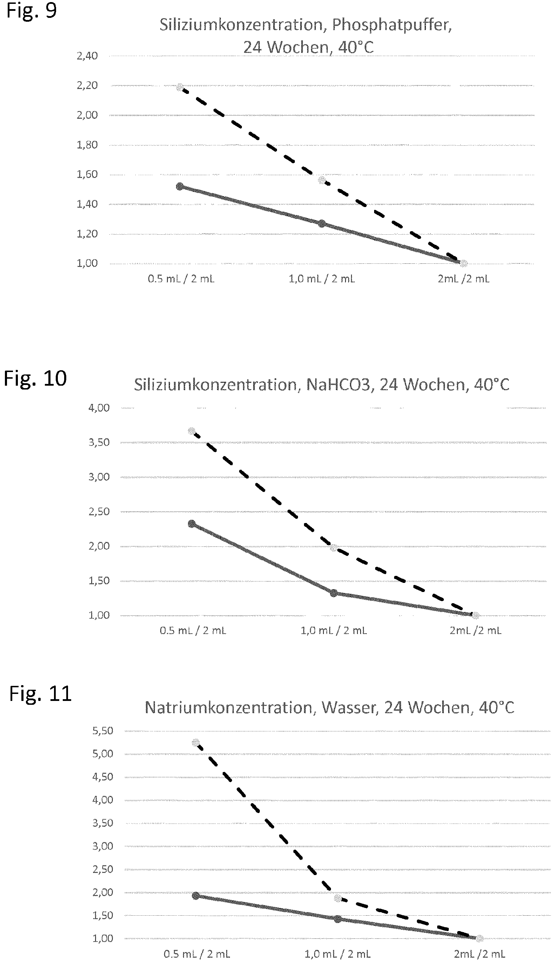

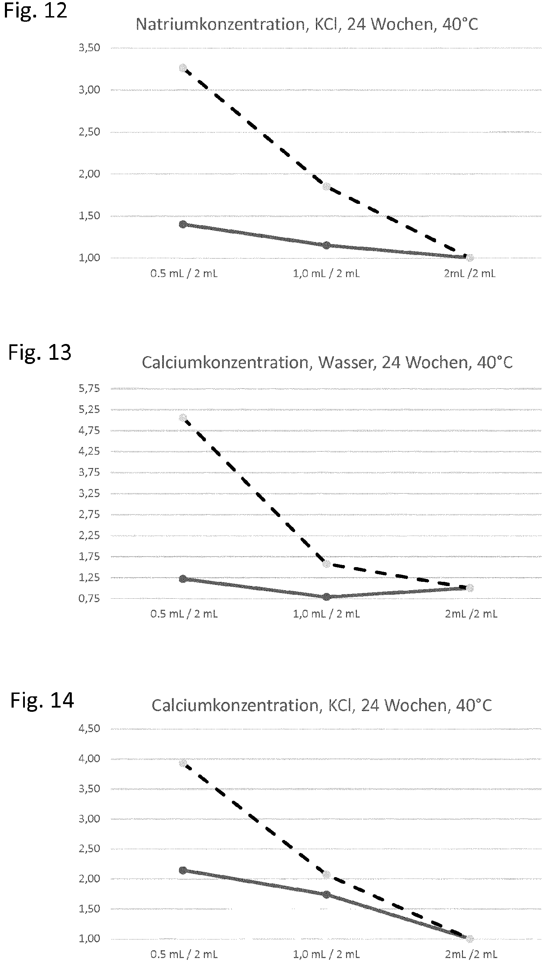

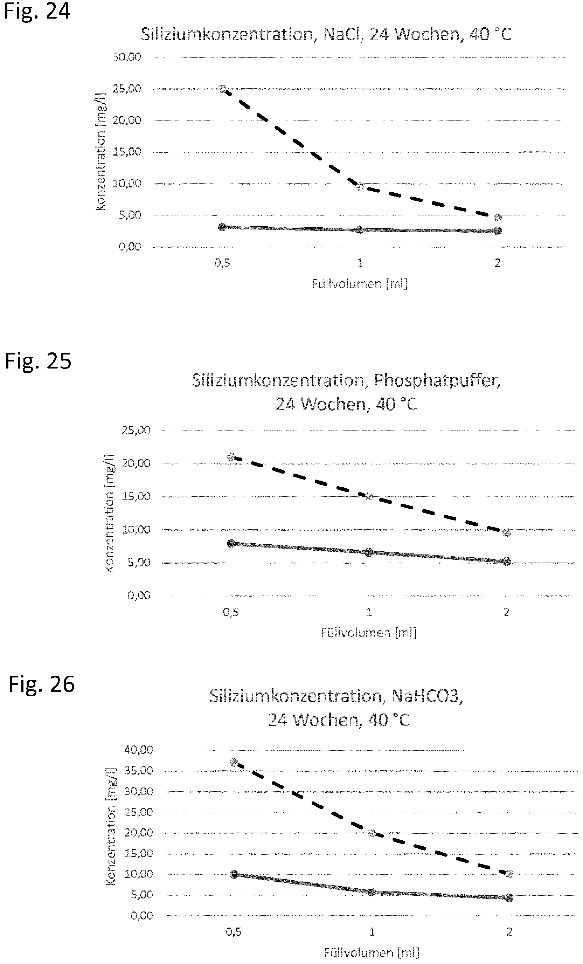

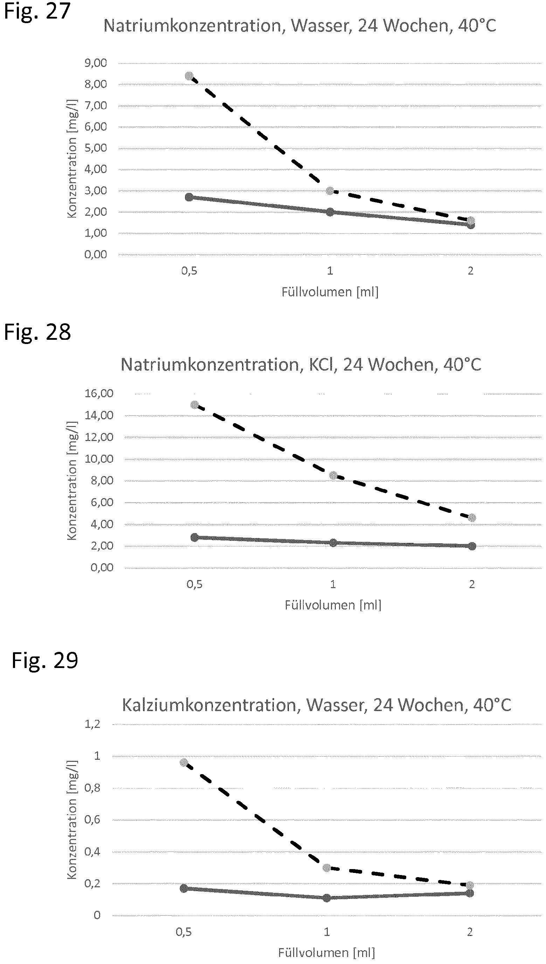

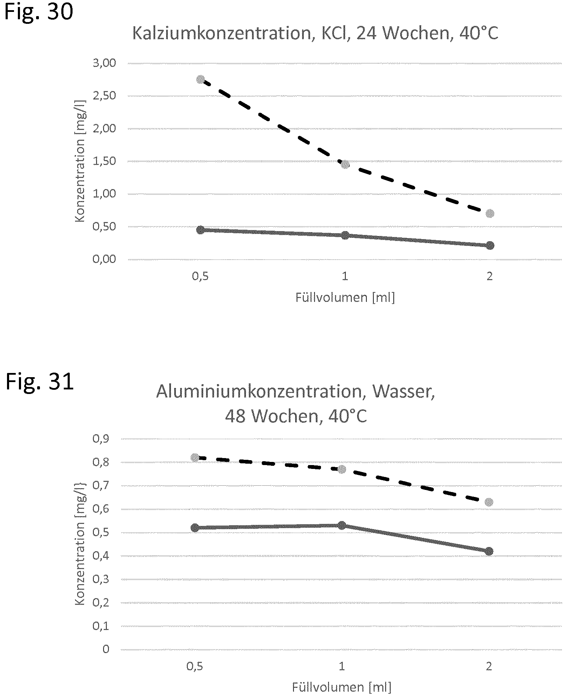

In den Fig. 2 bis 31 wird das Auslösungsverhalten eines Ausführungsbeispiels sowie eines Vergleichsbeispiels bezüglich verschiedener Glasbestandteile mit unterschiedlichen Flüssigkeiten als Auslösungsmedium dargestellt. Hierbei wird das entsprechende Auslösungsverhalten des Ausführungsbeispiels in den Diagrammen als durchgezogene Linie und das Auslösungsverhalten des Vergleichsbeispiels als gestrichelte Linie dargestellt.

Bei dem Vergleichsbeispiel handelt es sich um ein aus dem Stand der Technik bekannten Glasfläschchen, die als Pharmapackmittel verwendet werden. Sowohl Ausführungsbeispiel als auch Vergleichsbeispiel sind aus einem Neutralglas der Klasse I gefertigt. Das Nennvolumen der beiden Glasfläschchen betrug jeweils 2 ml.

Zur Ermittlung des Auslösungsverhaltens wurden drei verschiedene Füllvolumina, 0,5 ml, 1 ml und 2 ml mit unterschiedlichen Flüssigkeiten als Auslösungsmedium betrachtet. Die so gefüllten Glasfläschchen wurden jeweils für t1=24 Wochen und t2=48 Wochen bei 40°C gelagert. Nach Ablauf dieser Lagerzeiten wurden die Konzentrationen der verschiedenen ausgelösten, d.h. aus der Innenwandung 21 des Glasfläschchens in die Flüssigkeit übergegangenen Glasbestandteile mittels ICP-Verfahren gemessen. Hierbei wurde die Konzentration der Bestandteile Si, B, Al, Ca und in einigen Fällen Na bestimmt.

Nach Ablauf der Lagerzeiten wurden die Glasfläschchen verschiedenen Analyseverfahren unterworfen. Es wurden HR-ICP-MS (High Resolurion Inductively Coupled Plasma Mass Spectrometry) / ICP-OES (Inductively Coupled Plasma - Optical Emission Spectroscopy) Analysen durchgeführt, wobei jedes Füllvolumen (in doppelter Bestimmung) auf mindestens 5 ml jeden Satzes und beider Zugpunkte zusammengefasst wurde. Auf diese Weise wurden die Konzentrationen der in das Auslösemedium übergegangenen Glasbestandteile Si, B, Al, Ca und Na quantitativ bestimmt. Die Na-Konzentration wurde lediglich im Falle von Wasser und KCl als Füllmedium bestimmt

Das Ausführungsbeispiel und das Vergleichsbeispiel wurden mit folgenden Flüssigkeiten als Auslösemedium befüllt:
Probe 01: Aufbereitetes Wasser
Probe 02: Aufbereitetes Wasser mit Dampfsterilisation
Probe 03: Isotonische Kochsalzlösung (0,9%)
Probe 04: Isotonische Kochsalzlösung (0,9%) mit Dampfsterilisation
Probe 05: Phosphathaltige Pufferlösung mit PH-Wert 7
Probe 06: NaHCOs, 8,4%
Probe 07: KCI-Lösung, 15%
Anschließend wurden die Glasfläschchen mit einem Gummistopfen und einer Aluminiumkappe verschlossen.

Bei den Proben 2 und 5 wurde zusätzlich vor der Lagerung der Glasfläschchen eine Dampfsterilisation für 60 Minuten bei 121 °C durchgeführt. Bei allen Probe wurde während der Lagerung der Proben bei 40°C die Feuchtigkeit nicht geregelt.

Die Untersuchungsergebnisse werden in den folgenden Tabellen 6 bis 20 zusammengefasst.

Tabelle 6 zeigt die ICP Ergebnisse für die Auslösemedien vor der Befüllung.

**Tabelle 6: ICP Ergebnisse für die Auslösemedien vor der Befüllung**

| Blindlösung | B [mg/l] | Na [mg/l] | Al [mg/l] | Si [mg/l] | Ca [mg/l] |
|---|---|---|---|---|---|
| Aufbereitetes Wasser | < 0,005 | < 0,01 | < 0,005 | 0,008 ± 15% | < 0,005 |
| Bestimmungsgrenze | 0,005 | 0,01 | 0,005 | 0,005 | 0,005 |
| 0,9% NaCl | < 0,05 | - | < 0,05 | < 0,05 | < 0,05 |
| Bestimmungsgrenze | 0,05 | - | 0,05 | 0,05 | 0,05 |
| Phosphatpuffer | < 0,10 | - | < 0,10 | < 0,10 | < 0,10 |
| Bestimmungsgrenze | 0,10 | - | 0,10 | 0,10 | 0,10 |
| 8,4% NaHCO₃ | < 0,10 | - | < 0,10 | 1,4 ± 10% | 4,2 ± 10% |
| Bestimmungsgrenze | 0,10 | - | 0,10 | 0,50 | 1,25 |
| 15% KCI | < 0,20 | 1,3 ± 15% | < 0,20 | < 0,30 | < 0,20 |
| Bestimmungsgrenze | 0,20 | 0,20 | 0,20 | 0,30 | 0,20 |

Tabelle 7 zeigt die HR-ICP-MS Ergebnisse für aufbereitetes Wasser nach einer Lagerzeit von 24 Wochen.

**Tabelle 7: HR-ICP-MS Ergebnisse für aufbereitetes Wasser**

| Aufbereitetes Wasser | B | Na | Al | Si | Ca |
|---|---|---|---|---|---|
| Proben 01 | [mg/l] | [mg/l] | [mg/l] | [mg/l] | [mg/l] |
| Ausführungsbeispiel: 0,5 ml_A | 1,1 ± 10% | 2,6 ± 10% | 0,50 ± 10% | 5,2 ± 10% | 0,17 ± 10% |
| Ausführungsbeispiel: 0,5 ml_B | 1,1 ± 10% | 2,8 ± 10% | 0,52 ± 10% | 5,3 ± 10% | 0,10 ± 25% |
| Ausführungsbeispiel: 1,0 ml_A | 0,69 ± 10% | 1,9 ± 10% | 0,40 ± 10% | 4,5 ± 10% | 0,11 ± 10% |
| Ausführungsbeispiel: 1,0 ml_B | 0,72 ± 10% | 2,1 ± 10% | 0,53 ± 10% | 5,0 ± 10% | 0,13 ± 10% |
| Ausführungsbeispiel: 2,0 ml_A | 0,55 ± 10% | 1,5 ± 10% | 0,53 ± 10% | 4,5 ± 10% | 0,14 ± 10% |
| Ausführungsbeispiel: 2,0 ml_B | 0,49 ± 10% | 1,3 ± 10% | 0,42 ± 10% | 4,1 ± 10% | 0,12 ± 10% |
| Vergleichsbeispiel: 0,5 ml_A | 4,8 ± 10% | 9,8 ± 10% | 0,64 ± 10% | 16 ± 10% | 0,96 ± 10% |
| Vergleichsbeispiel: 0,5 ml_B | 3,3 ± 10% | 7,0 ± 10% | 0,82 ± 10% | 13 ± 10% | 0,57 ± 10% |
| Vergleichsbeispiel: 1,0 ml_A | 1,4 ± 10% | 3,2 ± 10% | 0,86 ± 10% | 7,8 ± 10% | 0,30 ± 10% |
| Vergleichsbeispiel: 1,0 ml_B | 1,2 ± 10% | 2,8 ± 10% | 0,77 ± 10% | 7,1 ± 10% | 0,27 ± 10% |
| Vergleichsbeispiel: 2,0 ml_A | 0,70 ± 10% | 1,7 ± 10% | 0,67 ± 10% | 5,2 ± 10% | 0,19 ± 25% |
| Vergleichsbeispiel: 2,0 ml_B | 0,65 ± 10% | 1,4 ± 10% | 0,63 ± 10% | 4,7 ± 10% | 0,18 ± 25% |
| Bestimmungsgrenze | 0,05 | 0,10 | 0,05 | 0,05 | 0,05 |

Tabelle 8 zeigt die HR-ICP-MS Ergebnisse für mit Wasser gefüllte und mit Dampf sterilisierte Proben nach einer Lagerzeit von 24 Wochen.

**Tabelle 8: HR-ICP-MS Ergebnisse für mit aufbereitetem Wasser gefüllte und mit Dampf sterilisierte Proben**

| Aufbereitetes Wasser | B | Na | Al | Si | Ca |
|---|---|---|---|---|---|
| Proben 02 | [mg/l] | [mg/l] | [mg/l] | [mg/l] | [mg/l] |
| Ausführungsbeispiel: 0,5 ml_A | 1,6 ± 10% | 4,0 ± 10% | 0,66 ± 10% | 8,3 ± 10% | 0,20 ± 10% |
| Ausführungsbeispiel: 0,5 ml_B | 1,5 ± 10% | 3,8 ± 10% | 0,67 ± 10% | 8,2 ± 10% | 0,18 ± 25% |
| Ausführungsbeispiel: 1,0 ml_A | 0,94 ± 10% | 2,6 ± 10% | 0,60 ± 10% | 6,5 ± 10% | 0,16 ± 10% |
| Ausführungsbeispiel: 1,0 ml_B | 0,93 ± 10% | 2,4 ± 10% | 0,51 ± 10% | 5,9 ± 10% | 0,12 ± 10% |
| Ausführungsbeispiel: 2,0 ml_A | 0,57 ± 10% | 1,8 ± 10% | 0,57 ± 10% | 5,0 ± 10% | 0,14 ± 10% |
| Ausführungsbeispiel: 2,0 ml_B | 0,65 ± 10% | 1,8 ± 10% | 0,60 ± 10% | 5,3 ± 10% | 0,15 ± 10% |
| Vergleichsbeispiel: 0,5 ml_A | 5,2 ± 10% | 9,9 ± 10% | 1,0 ± 10% | 20 ±10% | 0,89 ± 10% |
| Vergleichsbeispiel: 0,5 ml_B | 4,8 ± 10% | 9,5 ± 10% | 0,97 ± 10% | 19 ± 10% | 0,96 ± 10% |
| Vergleichsbeispiel: 1,0 ml_A | 1,9 ± 10% | 3,9 ± 10% | 0,81 ± 10% | 11 ± 10% | 0,24 ± 10% |
| Vergleichsbeispiel: 1,0 ml_B | 1,8 ± 10% | 3,7 ± 10% | 0,74 ± 10% | 10 ± 10% | 0,24 ± 10% |
| Vergleichsbeispiel: 2,0 ml_A | 1,0 ± 10% | 2,3 ± 10% | 0,65 ± 10% | 7,1 ± 10% | 0,21 ± 10% |
| Vergleichsbeispiel: 2,0 ml_B | 1,0 ± 10% | 2,3 ± 10% | 0,64 ± 10% | 6,9 ± 10% | 0,21 ± 10% |
| Bestimmungsgrenze | 0,05 | 0,10 | 0,05 | 0,05 | 0,05 |

Tabelle 9 zeigt die HR-ICP-MS Ergebnisse für mit 0,9% NaCl gefüllte Proben nach einer Lagerzeit von 24 Wochen, wobei die relativen Messfehler mit k = 2 berechnet wurden.

**Tabelle 9: HR-ICP-MS Ergebnisse für mit 0,9% NaCl gefüllte Proben**

| 0,9% NaCl | B | Al | Si | Ca |
|---|---|---|---|---|
| Proben 03 | [mg/l] | [mg/l] | [mg/l] | [mg/l] |
| Ausführungsbeispiel: 0,5 ml_A | 1,2 ± 10% | 0,76 ± 10% | 3,0 ± 10% | 0,46 ± 10% |
| Ausführungsbeispiel: 0,5 ml_B | 1,1 ± 10% | 0,11 ± 10% | 3,2 ± 10% | 0,46 ± 10% |
| Ausführungsbeispiel: 1,0 ml_A | 0,67 ± 10% | 0,13 ± 10% | 2,5 ± 10% | 0,29 ± 10% |
| Ausführungsbeispiel: 1,0 ml_B | 0,67 ± 10% | 0,14 ± 10% | 2,8 ± 10% | 0,29 ± 10% |
| Ausführungsbeispiel: 2,0 ml_A | 0,49 ± 10% | 0,16 ± 10% | 2,4 ± 10% | 0,23 ± 10% |
| Ausführungsbeispiel: 2,0 ml_B | 0,50 ± 10% | 0,17 ± 10% | 2,5 ± 10% | 0,21 ± 10% |
| Vergleichsbeispiel: 0,5 ml_A | 8,4 ± 10% | < 0,10 | 25 ± 10% | 3,9 ± 10% |
| Vergleichsbeispiel: 0,5 ml_B | 8,3 ± 10% | < 0,10 | 24 ± 10% | 2,6 ± 10% |
| Vergleichsbeispiel: 1,0 ml_A | 2,8 ± 10% | < 0,10 | 9,7 ± 10% | 0,89 ± 10% |
| Vergleichsbeispiel: 1,0 ml_B | 2,8 ± 10% | < 0,10 | 9,3 ± 10% | 0,87 ± 10% |
| Vergleichsbeispiel: 2,0 ml_A | 1,2 ± 10% | < 0,10 | 4,9 ± 10% | 0,41 ± 10% |
| Vergleichsbeispiel: 2,0 ml_B | 1,1 ± 10% | < 0,10 | 4,5 ± 10% | 0,39 ± 10% |
| Bestimmungsgrenze | 0,10 | 0,10 | 0,10 | 0,10 |

Tabelle 10 zeigt die HR-ICP-MS Ergebnisse für mit 0,9% NaCl gefüllte und mit Dampf sterilisierte Proben nach einer Lagerzeit von 24 Wochen.

**Tabelle 10: HR-ICP-MS Ergebnisse für mit 0,9% NaCl gefüllte und mit Dampf sterilisierte Proben**

| 0,9% NaCl | B | Al | Si | Ca |
|---|---|---|---|---|
| Proben 04 | [mg/l] | [mg/l] | [mg/l] | [mg/l] |
| Ausführungsbeispiel: 0,5 ml_A | 1,2 ± 10% | < 0,10 | 4,7 ± 10% | 0,69 ± 10% |
| Ausführungsbeispiel: 0,5 ml_B | 1,2 ± 10% | < 0,10 | 4,6 ± 10% | 0,62 ± 10% |
| Ausführungsbeispiel: 1,0 ml_A | 0,58 ± 10% | < 0,10 | 3,1 ± 10% | 0,34 ± 10% |
| Ausführungsbeispiel: 1,0 ml_B | 0,62 ± 10% | < 0,10 | 3,2 ± 10% | 0,34 ± 10% |
| Ausführungsbeispiel: 2,0 ml_A | 0,34 ± 10% | < 0,10 | 2,4 ± 10% | 0,22 ± 25% |
| Ausführungsbeispiel: 2,0 ml_B | 0,34 ± 10% | < 0,10 | 2,3 ± 10% | 0,20 ± 25% |
| Vergleichsbeispiel: 0,5 ml_A | 4,0 ± 10% | 0,16 ± 10% | 14 ± 10% | 1,7 ± 10% |
| Vergleichsbeispiel: 0,5 ml_B | 4,3 ± 10% | 0,29 ± 10% | 15 ± 10% | 2,9 ± 10% |
| Vergleichsbeispiel: 1,0 ml_A | 2,3 ± 10% | 0,16 ± 10% | 9,3 ± 10% | 0,82 ± 10% |
| Vergleichsbeispiel: 1,0 ml_B | 2,3 ± 10% | 0,14 ± 10% | 9,2 ± 10% | 0,80 ± 10% |
| Vergleichsbeispiel: 2,0 ml_A | 1,4 ± 10% | 0,16 ± 10% | 6,6 ± 10% | 0,50 ± 10% |
| Vergleichsbeispiel: 2,0 ml_B | 1,4 ± 10% | 0,14 ± 10% | 6,4 ± 10% | 0,52 ± 10% |
| Bestimmungsgrenze | 0,10 | 0,10 | 0,10 | 0,10 |

Tabelle 11 zeigt die HR-ICP-MS Ergebnisse für mit einer Phosphatpufferlösung gefüllte Proben nach einer Lagerzeit von 24 Wochen.

**Tabelle 11: HR-ICP-MS Ergebnisse für mit einer Phosphatpufferlösung gefüllte Proben**

| Phosphatpufferlösung | B | Al | Si | Ca |
|---|---|---|---|---|
| Proben 05 | [mg/l] | [mg/l] | [mg/l] | [mg/l] |
| Ausführungsbeispiel: 0,5 ml_A | 1,5 ± 10% | < 0,20 | 7,8 ± 10% | 0,61 ± 10% |
| Ausführungsbeispiel: 0,5 ml_B | 1,6 ± 10% | < 0,20 | 7,9 ± 10% | 0,63 ± 10% |
| Ausführungsbeispiel: 1,0 ml_A | 1,0 ± 10% | < 0,20 | 6,4 ± 10% | 0,42 ± 10% |
| Ausführungsbeispiel: 1,0 ml_B | 1,1 ± 10% | < 0,20 | 6,7 ± 10% | 0,43 ± 10% |
| Ausführungsbeispiel: 2,0 ml_A | 0,66 ± 10% | < 0,20 | 5,1 ± 10% | 0,30 ± 25% |
| Ausführungsbeispiel: 2,0 ml_B | 0,68 ± 10% | < 0,20 | 5,2 ± 10% | 0,27 ± 25% |
| Vergleichsbeispiel: 0,5 ml_A | 6,5 ± 10% | < 0,20 | 21 ± 10% | 2,5 ± 10% |
| Vergleichsbeispiel: 0,5 ml_B | 6,6 ± 10% | < 0,20 | 21 ± 10% | 2,4 ± 10% |
| Vergleichsbeispiel: 1,0 ml_A | 3,2 ± 10% | < 0,20 | 15 ± 10% | 0,97 ± 10% |
| Vergleichsbeispiel: 1,0 ml_B | 3,0 ± 10% | < 0,20 | 14 ± 10% | 0,84 ± 10% |
| Vergleichsbeispiel: 2,0 ml_A | 1,6 ± 10% | < 0,20 | 9,5 ± 10% | 0,53 ± 10% |
| Vergleichsbeispiel: 2,0 ml_B | 1,6 ± 10% | < 0,20 | 9,6 ± 10% | 0,51 ± 10% |
| Bestimmungsgrenze | 0,20 | 0,20 | 0,20 | 0,20 |

Tabelle 12 zeigt die HR-ICP-MS Ergebnisse für mit 8,4% NaHCOs gefüllte Proben nach einer Lagerzeit von 24 Wochen.

**Tabelle 12: HR-ICP-MS Ergebnisse für mit 8,4% NaHCOs gefüllte Proben**

| 8,4% NaHCO₃ | B | Al | Si | Ca |
|---|---|---|---|---|
| Proben 06 | [mg/l] | [mg/l] | [mg/l] | [mg/l] |
| Ausführungsbeispiel: 0,5 ml_A | 2,1 ±10% | < 0,20 | 10 ± 10% | 2,5 ± 10% |
| Ausführungsbeispiel: 0,5 ml_B | 2,0 ± 10% | < 0,20 | 10 ± 10% | 2,2 ± 10% |
| Ausführungsbeispiel: 1,0 ml_A | 0,94 ± 10% | < 0,20 | 5,7 ± 10% | 4,6 ± 10% |
| Ausführungsbeispiel: 1,0 ml_B | 0,93 ± 10% | < 0,20 | 5,7 ± 10% | 4,1 ± 10% |
| Ausführungsbeispiel: 2,0 ml_A | 0,47 ± 10% | 0,20 ± 10% | 4,2 ± 10% | 5,0 ± 10% |
| Ausführungsbeispiel: 2,0 ml_B | 0,49 ± 10% | 0,20 ± 10% | 4,3 ± 10% | 5,0 ± 10% |
| Vergleichsbeispiel: 0,5 ml_A | 9,4 ± 10% | < 0,20 | 37 ± 10% | 1,9 ± 10% |
| Vergleichsbeispiel: 0,5 ml_B | 8,9 ± 10% | < 0,20 | 36 ± 10% | 1,8 ± 10% |
| Vergleichsbeispiel: 1,0 ml_A | 4,3 ± 10% | < 0,20 | 19 ± 10% | 5,9 ± 10% |
| Vergleichsbeispiel: 1,0 ml_B | 4,4 ± 10% | < 0,20 | 20 ± 10% | 5,6 ± 10% |
| Vergleichsbeispiel: 2,0 ml_A | 2,0 ± 10% | < 0,20 | 10 ± 10% | 5,4 ± 10% |
| Vergleichsbeispiel: 2,0 ml_B | 2,0 ± 10% | < 0,20 | 10 ± 10% | 5,4 ± 10% |
| Bestimmungsgrenze | 0,20 | 0,20 | 0,50 | 1,25 |

Tabelle 13 zeigt die ICP-OES Ergebnisse für mit 15% KCl gefüllte Proben nach einer Lagerzeit von 24 Wochen.

**Tabelle 13: ICP-OES Ergebnisse für mit 15% KCL gefüllte Proben**

| 15% KCl | B | Na | Al | Si | Ca |
|---|---|---|---|---|---|
| Proben 07 | [mg/l] | [mg/l] | [mg/l] | [mg/l] | [mg/l] |
| Ausführungsbeispiel: 0,5 ml_A | 0,91 ± 15% | 2,9 ± 15% | < 0,20 | 1,4 ± 15% | 0,45 ± 30% |
| Ausführungsbeispiel: 0,5 ml_B | 0,92 ± 15% | 2,7 ± 15% | < 0,20 | 1,3 ± 15% | 0,44 ± 30% |
| Ausführungsbeispiel: 1,0 ml_A | 0,65 ± 15% | 2,3 ± 15% | < 0,20 | 1,3 ± 15% | 0,37 ± 30% |
| Ausführungsbeispiel: 1,0 ml_B | 0,59 ± 15% | 2,2 ± 15% | < 0,20 | 1,2 ± 15% | 0,36 ± 30% |
| Ausführungsbeispiel: 2,0 ml_A | 0,35 ± 30% | 1,9 ± 15% | < 0,20 | 1,1 ± 15% | 0,21 ± 30% |
| Ausführungsbeispiel: 2,0 ml_B | 0,33 ± 30% | 2,1 ± 15% | < 0,20 | 1,1 ± 15% | 0,21 ± 30% |
| Vergleichsbeispiel: 0,5 ml_A | 8,0 ± 10% | 15 ± 10% | < 0,20 | 23 ± 10% | 2,7 ± 15% |
| Vergleichsbeispiel: 0,5 ml_B | 8,4 ± 10% | 15 ± 10% | < 0,20 | 24 ± 10% | 2,8 ± 15% |
| Vergleichsbeispiel: 1,0 ml_A | 4,5 ± 15% | 8,3 ± 10% | < 0,20 | 15 ± 10% | 1,5 ± 15% |
| Vergleichsbeispiel: 1,0 ml_B | 4,6 ± 15% | 8,7 ± 10% | < 0,20 | 16 ± 10% | 1,4 ± 15% |
| Vergleichsbeispiel: 2,0 ml_A | 2,2 ± 15% | 4,8 ± 15% | < 0,20 | 8,5 ± 10% | 0,74 ± 15% |
| Vergleichsbeispiel: 2,0 ml_B | 1,9 ± 15% | 4,3 ± 15% | < 0,20 | 7,5 ± 10% | 0,66 ± 15% |
| Bestimmungsgrenze | 0,20 | 0,20 | 0,20 | 0,30 | 0,20 |

Tabelle 14 zeigt die HR-ICP-MS Ergebnisse für aufbereitetes Wasser nach einer Lagerzeit von 48 Wochen.

**Tabelle 14: HR-ICP-MS Ergebnisse für mit aufbereitetem Wasser befüllte Proben**

| Aufbereitetes Wasser | B | Na | Al | Si | Ca |
|---|---|---|---|---|---|
| Proben 11 | [mg/l] | [mg/l] | [mg/l] | [mg/l] | [mg/l] |
| Ausführungsbeispiel: 0,5 ml_A | 1,4 ± 10% | 3,3 ± 10% | 0,77 ± 10% | 8,2 ± 10% | 0,12 ± 25% |
| Ausführungsbeispiel: 0,5 ml_B | 1,4 ± 10% | 3,6 ± 10% | 0,83 ± 10% | 9,3 ± 10% | 0,16 ± 25% |
| Ausführungsbeispiel: 1,0 ml_A | 0,94 ± 10% | 2,4 ± 10% | 0,68 ± 10% | 6,6 ± 10% | 0,11 ± 25% |
| Ausführungsbeispiel: 1,0 ml_B | 0,85 ± 10% | 2,2 ± 10% | 0,57 ± 10% | 6,0 ± 10% | 0,13 ± 25% |
| Ausführungsbeispiel: 2,0 ml_A | 0,61 ± 10% | 1,6 ± 10% | 0,64 ± 10% | 5,5 ± 10% | 0,16 ± 25% |
| Ausführungsbeispiel: 2,0 ml_B | 0,65 ± 10% | 1,7 ± 10% | 0,72 ± 10% | 5,9 ± 10% | 0,16 ± 25% |
| Vergleichsbeispiel: 0,5 ml_A | 4,8 ± 10% | 8,7 ± 10% | 0,85 ± 10% | 17 ± 10% | 0,21 ± 25% |
| Vergleichsbeispiel: 0,5 ml_B | 5,1 ± 10% | 9,3 ± 10% | 0,71 ± 10% | 19 ± 10% | 0,24 ± 25% |
| Vergleichsbeispiel: 1,0 ml_A | 1,5 ± 10% | 3,3 ± 10% | 0,78 ± 10% | 9,3 ± 10% | 0,18 ± 25% |
| Vergleichsbeispiel: 1,0 ml_B | 1,4 ± 10% | 3,3 ± 10% | 0,67 ± 10% | 9,1 ± 10% | 0,14 ± 25% |
| Vergleichsbeispiel: 2,0 ml_A | 0,82 ± 10% | 2,0 ± 10% | 0,46 ± 10% | 6,0 ± 10% | 0,15 ± 25% |
| Vergleichsbeispiel: 2,0 ml_B | 0,78 ± 10% | 1,9 ± 10% | 0,41 ± 10% | 5,8 ± 10% | 0,14 ± 25% |
| Bestimmungsgrenze | 0,05 | 0,10 | 0,05 | 0,05 | 0,05 |

Tabelle 15 zeigt die HR-ICP-MS Ergebnisse für mit Wasser gefüllte und mit Dampf sterilisierte Proben nach einer Lagerzeit von 48 Wochen.

**Tabelle 15: HR-ICP-MS Ergebnisse für mit aufbereitetem Wasser gefüllte und mit Dampf sterilisierte Proben**

| Aufbereitetes Wasser | B | Na | Al | Si | Ca |
|---|---|---|---|---|---|
| Proben 12 | [mg/l] | [mg/l] | [mg/l] | [mg/l] | [mg/l] |
| Ausführungsbeispiel: 0,5 ml_A | 1,4 ± 10% | 3,8 ± 10% | 0,71 ± 10% | 10 ± 10% | 0,13 ± 25% |
| Ausführungsbeispiel: 0,5 ml_B | 1,5 ± 10% | 3,6 ± 10% | 0,61 ± 10% | 9,4 ±10% | 0,13 ± 25% |
| Ausführungsbeispiel: 1,0 ml_A | 1,0 ± 10% | 2,5 ± 10% | 0,57 ± 10% | 7,3 ±10% | 0,13 ± 25% |
| Ausführungsbeispiel: 1,0 ml_B | 1,1 ± 10% | 2,6 ± 10% | 0,52 ± 10% | 7,2 ± 10% | 0,12 ± 25% |
| Ausführungsbeispiel: 2,0 ml_A | 0,68 ± 10% | 1,8 ± 10% | 0,58 ± 10% | 5,9 ± 10% | 0,15 ± 25% |
| Ausführungsbeispiel: 2,0 ml_B | 0,69 ± 10% | 1,8 ± 10% | 0,63 ± 10% | 6,1 ± 10% | 0,14 ± 25% |
| Vergleichsbeispiel: 0,5 ml_A | 5,5 ± 10% | 10 ± 10% | 0,67 ± 10% | 21 ± 10% | 1,1 ± 10% |
| Vergleichsbeispiel: 0,5 ml_B | 5,4 ± 10% | 10 ± 10% | 0,80 ± 10% | 21 ± 10% | 0,89 ± 10% |
| Vergleichsbeispiel: 1,0 ml_A | 2,2 ± 10% | 4,4 ± 10% | 0,72 ± 10% | 13 ± 10% | 0,15 ± 25% |
| Vergleichsbeispiel: 1,0 ml_B | 2,4 ± 10% | 4,6 ± 10% | 0,83 ± 10% | 12 ± 10% | 0,12 ± 25% |
| Vergleichsbeispiel: 2,0 ml_A | 1,0 ± 10% | 2,4 ± 10% | 0,52 ± 10% | 8,3 ± 10% | 0,16 ± 25% |
| Vergleichsbeispiel: 2,0 ml_B | 1,0 ± 10% | 2,3 ± 10% | 0,46 ± 10% | 7,9 ± 10% | 0,15 ± 25% |
| Bestimmungsgrenze | 0,05 | 0,10 | 0,05 | 0,05 | 0,05 |

Tabelle 16 zeigt die HR-ICP-MS Ergebnisse für mit 0,9% NaCl gefüllte Proben nach einer Lagerzeit von 48 Wochen.

**Tabelle 16: HR-ICP-MS Ergebnisse für mit 0,9% NaCl gefüllte Proben**

| 0,9% NaCl | B | Al | Si | Ca |
|---|---|---|---|---|
| Proben 13 | [mg/l] | [mg/l] | [mg/l] | [mg/l] |
| Ausführungsbeispiel: 0,5 ml_A | 1,3 ± 10% | 0,10 ± 10% | 4,3 ± 10% | 0,48 ± 10% |
| Ausführungsbeispiel: 0,5 ml_B | 1,2 ± 10% | 0,10 ± 10% | 4,2 ± 10% | 0,47 ± 10% |
| Ausführungsbeispiel: 1,0 ml_A | 0,65 ± 10% | 0,14 ± 10% | 3,3 ± 10% | 0,30 ± 10% |
| Ausführungsbeispiel: 1,0 ml_B | 0,64 ± 10% | 0,14 ± 10% | 3,3 ± 10% | 0,32 ± 10% |
| Ausführungsbeispiel: 2,0 ml_A | 0,30 ± 10% | 0,19 ± 10% | 2,6 ± 10% | 0,18 ± 10% |
| Ausführungsbeispiel: 2,0 ml_B | 0,32 ± 10% | 0,21 ± 10% | 2,8 ± 10% | 0,17 ± 10% |
| Vergleichsbeispiel: 0,5 ml_A | 11 ± 10% | < 0,10 | 33 ± 10% | 4,3 ± 10% |
| Vergleichsbeispiel: 0,5 ml_B | 11 ± 10% | < 0,10 | 33 ± 10% | 4,2 ± 10% |
| Vergleichsbeispiel: 1,0 ml_A | 2,7 ± 10% | < 0,10 | 11 ± 10% | 0,96 ± 10% |
| Vergleichsbeispiel: 1,0 ml_B | 3,4 ± 10% | < 0,10 | 14 ± 10% | 1,1 ± 10% |
| Vergleichsbeispiel: 2,0 ml_A | 1,4 ± 10% | 0,13 ± 10% | 6,7 ± 10% | 0,52 ± 10% |
| Vergleichsbeispiel: 2,0 ml_B | 1,4 ± 10% | 0,14 ± 10% | 6,2 ± 10% | 0,50 ± 10% |
| Bestimmungsgrenze | 0,10 | 0,10 | 0,10 | 0,10 |

Tabelle 17 zeigt die HR-ICP-MS Ergebnisse für mit 0,9% NaCl gefüllte und mit Dampf sterilisierte Proben bei nach einer Lagerzeit von 48 Wochen, wobei die relativen Messfehler mit k = 2 berechnet wurden.

**Tabelle 17: HR-ICP-MS Ergebnisse für mit 0,9% NaCl gefüllte und mit Dampf sterilisierte Proben**

| 0,9% NaCl | B | Al | Si | Ca |
|---|---|---|---|---|
| Proben 14 | [mg/l] | [mg/l] | [mg/l] | [mg/l] |
| Ausführungsbeispiel: 0,5 ml_A | 1,5 ± 10% | 0,10 ± 10% | 6,9 ± 10% | 0,81 ± 10% |
| Ausführungsbeispiel: 0,5 ml_B | 1,5 ± 10% | 0,11 ± 10% | 6,6 ± 10% | 0,79 ± 10% |
| Ausführungsbeispiel: 1,0 ml_A | 0,72 ± 10% | 0,11 ± 10% | 4,2 ± 10% | 0,40 ± 10% |
| Ausführungsbeispiel: 1,0 ml_B | 0,73 ± 10% | 0,10 ± 10% | 4,0 ± 10% | 0,43 ± 10% |
| Ausführungsbeispiel: 2,0 ml_A | 0,37 ± 10% | 0,17 ± 10% | 3,3 ± 10% | 0,21 ± 10% |
| Ausführungsbeispiel: 2,0 ml_B | 0,34 ± 10% | 0,17 ± 10% | 3,2 ± 10% | 0,20 ± 10% |
| Vergleichsbeispiel: 0,5 ml_A | 4,9 ± 10% | 0,23 ± 10% | 17 ± 10% | 2,3 ± 10% |
| Vergleichsbeispiel: 0,5 ml_B | 5,1 ± 10% | 0,21 ± 10% | 17 ± 10% | 2,3 ± 10% |
| Vergleichsbeispiel: 1,0 ml_A | 3,0 ± 10% | < 0,10 | 12 ± 10% | 1,0 ± 10% |
| Vergleichsbeispiel: 1,0 ml_B | 2,9 ± 10% | 0,12 ± 10% | 12 ± 10% | 0,97 ± 10% |
| Vergleichsbeispiel: 2,0 ml_A | 1,9 ± 10% | 0,12 ± 10% | 8,8 ± 10% | 0,64 ± 10% |
| Vergleichsbeispiel: 2,0 ml_B | 1,8 ± 10% | 0,16 ± 10% | 8,8 ± 10% | 0,62 ± 10% |
| Bestimmungsgrenze | 0,10 | 0,10 | 0,10 | 0,10 |

Tabelle 18 zeigt die HR-ICP-MS Ergebnisse für mit einer Phosphatpufferlösung gefüllte Proben nach einer Lagerzeit von 48 Wochen.

**Tabelle 18: HR-ICP-MS Ergebnisse für mit einer Phosphatpufferlösung gefüllte Proben**

| Phosphatpufferlösung | B | Al | Si | Ca |
|---|---|---|---|---|
| Proben 15 | [mg/l] | [mg/l] | [mg/l] | [mg/l] |
| Ausführungsbeispiel: 0,5 ml_A | 1,8 ± 10% | < 0,20 | 12 ± 10% | 0,67 ± 10% |
| Ausführungsbeispiel: 0,5 ml_B | 1,7 ± 10% | < 0,20 | 12 ± 10% | 0,69 ± 10% |
| Ausführungsbeispiel: 1,0 ml_A | 1,2 ± 10% | < 0,20 | 11 ± 10% | 0,48 ± 10% |
| Ausführungsbeispiel: 1,0 ml_B | 1,1 ± 10% | < 0,20 | 10 ± 10% | 0,46 ± 10% |
| Ausführungsbeispiel: 2,0 ml_A | 0,82 ± 10% | < 0,20 | 8,5 ± 10% | 0,73 ± 10% |
| Ausführungsbeispiel: 2,0 ml_B | 0,79 ± 10% | < 0,20 | 8,6 ± 10% | 0,34 ± 25% |
| Vergleichsbeispiel: 0,5 ml_A | 6,4 ± 10% | < 0,20 | 27 ± 10% | 2,3 ± 10% |
| Vergleichsbeispiel: 0,5 ml_B | 6,0 ± 10% | < 0,20 | 26 ± 10% | 2,6 ± 10% |
| Vergleichsbeispiel: 1,0 ml_A | 2,9 ± 10% | < 0,20 | 19 ± 10% | 0,92 ± 10% |
| Vergleichsbeispiel: 1,0 ml_B | 3,0 ± 10% | < 0,20 | 18 ± 10% | 0,92 ± 10% |
| Vergleichsbeispiel: 2,0 ml_A | 1,6 ± 10% | < 0,20 | 13 ± 10% | 0,52 ± 10% |
| Vergleichsbeispiel: 2,0 ml_B | 1,6 ± 10% | < 0,20 | 13 ± 10% | 0,53 ± 10% |
| Bestimmungsgrenze | 0,20 | 0,20 | 0,20 | 0,20 |

Tabelle 19 zeigt die HR-ICP-MS Ergebnisse für mit 8,4% NaHCOs gefüllte Proben nach einer Lagerzeit von 48 Wochen, wobei die relativen Messfehler mit k = 2 berechnet wurden.

**Tabelle 19: HR-ICP-MS Ergebnisse für mit 8,4% NaHCOs gefüllte Proben**

| 8,4%NaHCO₃ | B | Al | Si | Ca |
|---|---|---|---|---|
| Proben 16 | [mg/l] | [mg/l] | [mg/l] | [mg/l] |
| Ausführungsbeispiel: 0,5 ml_A | 3,2 ± 10% | < 0,20 | 20 ± 10% | 2,2 ± 10% |
| Ausführungsbeispiel: 0,5 ml_B | 3,5 ± 10% | < 0,20 | 20 ± 10% | 2,2 ± 10% |
| Ausführungsbeispiel: 1,0 ml_A | 1,3 ± 10% | < 0,20 | 9,0 ± 10% | 2,4 ± 10% |
| Ausführungsbeispiel: 1,0 ml_B | 1,3 ± 10% | < 0,20 | 8,9 ± 10% | 3,0 ± 10% |
| Ausführungsbeispiel: 2,0 ml_A | 0,67 ± 10% | < 0,20 | 5,7 ± 10% | 5,0 ± 10% |
| Ausführungsbeispiel: 2,0 ml_B | 0,69 ± 10% | < 0,20 | 5,9 ± 10% | 5,3 ± 10% |
| Vergleichsbeispiel: 0,5 ml_A | 11 ± 10% | < 0,20 | 51 ± 10% | 2,1 ± 10% |
| Vergleichsbeispiel: 0,5 ml_B | 13 ± 10% | < 0,20 | 52 ± 10% | 2,0 ± 10% |
| Vergleichsbeispiel: 1,0 ml_A | 5,5 ± 10% | < 0,20 | 32 ± 10% | 2,1 ± 10% |
| Vergleichsbeispiel: 1,0 ml_B | 5,8 ± 10% | < 0,20 | 30 ± 10% | 2,0 ± 10% |
| Vergleichsbeispiel: 2,0 ml_A | 2,5 ± 10% | < 0,20 | 15 ± 10% | 5,5 ± 10% |
| Vergleichsbeispiel: 2,0 ml_B | 2,2 ± 10% | < 0,20 | 15 ± 10% | 5,6 ± 10% |
| Bestimmungsgrenze | 0,20 | 0,20 | 0,50 | 1,25 |

Tabelle 20 zeigt die ICP-OES Ergebnisse für mit 15% KCl gefüllte Proben nach einer Lagerzeit von 48 Wochen, wobei die relativen Messfehler mit k = 2 berechnet wurden.

**Tabelle 20: ICP-OES Ergebnisse für mit 15% KCL gefüllte Proben**

| 15% KCl | B | Na | Al | Si | Ca |
|---|---|---|---|---|---|
| Proben 17 | [mg/l] | [mg/l] | [mg/l] | [mg/l] | [mg/l] |
| Ausführungsbeispiel: 0,5 ml_A | 1,1 ± 15% | 3,6 ± 15% | < 0,20 | 1,8 ± 15% | 0,48 ± 30% |
| Ausführungsbeispiel: 0,5 ml_B | 1,1 ± 15% | 3,3 ± 15% | < 0,20 | 1,7 ± 15% | 0,47 ± 30% |
| Ausführungsbeispiel: 1,0 ml_A | 0,66 ± 15% | 2,4 ± 15% | < 0,20 | 1,3 ± 15% | 0,33 ± 30% |
| Ausführungsbeispiel: 1,0 ml_B | 0,66 ± 15% | 2,4 ± 15% | < 0,20 | 1,3 ± 15% | 0,32 ± 30% |
| Ausführungsbeispiel: 2,0 ml_A | 0,42 ± 30% | 1,9 ± 15% | < 0,20 | 1,2 ± 15% | < 0,20 |
| Ausführungsbeispiel: 2,0 ml_B | 0,42 ± 30% | 1,9 ± 15% | < 0,20 | 1,2 ± 15% | < 0,20 |
| Vergleichsbeispiel: 0,5 ml_A | 7,6 ± 10% | 15 ± 10% | < 0,20 | 23 ± 10% | 2,4 ± 15% |
| Vergleichsbeispiel: 0,5 ml_B | 7,7 ± 1 0% | 15 ± 10% | < 0,20 | 24 ± 10% | 2,5 ± 15% |
| Vergleichsbeispiel: 1,0 ml_A | 4,3 ± 15% | 8,6 ± 10% | < 0,20 | 16 ± 10% | 1,5 ± 15% |
| Vergleichsbeispiel: 1,0 ml_B | 4,4 ± 15% | 8,7 ± 10% | < 0,20 | 16 ± 10% | 1,4 ± 15% |
| Vergleichsbeispiel: 2,0 ml_A | 2,5 ± 15% | 5,2 ± 10% | < 0,20 | 10 ± 10% | 0,75 ± 15% |
| Vergleichsbeispiel: 2,0 ml_B | 2,6 ± 15% | 5,3 ± 10% | < 0,20 | 11 ± 10% | 0,76 ± 15% |
| Bestimmungsgrenze | 0,20 | 0,20 | 0,20 | 0,30 | 0,20 |

Die Fig. 2 bis 5 zeigen das Auslösungsverhalten von Ausführungsbeispiel und Vergleichsbeispiel für Bor bei unterschiedlichen Auslösungsmedien. Hierbei wird in den entsprechenden Diagrammen der Quotient der Konzentrationen beim dem jeweiligen Füllvolumen (0,5 ml, 1 ml und 2 ml) und bei der Konzentration bei einem Füllvolumen von 2 ml aufgetragen. Bei einem Füllvolumen von 0,5 ml wird im Wesentlichen der Boden des Glasfläschchens sowie die bodennahen Wandbereiche mit der Flüssigkeit benetzt und tragen somit zur Migrationslast bei. Die geringere Migrationslast von Wandbereichen mit einem größeren Abstand zum Boden geht dagegen nicht ein. Somit repräsentieren die Konzentrationen bei einem Füllvolumen von 0,5 ml das Auslöseverhalten vom Boden und den bodennahen Wandbereichen und somit die Migrationslast bei sehr geringen Füllungsgraden.

Bei einem Füllvolumen von 1 ml werden auch Wandbereiche mit einer geringeren Migrationslast bedeckt, so dass diese in die Gesamtmigrationslast eingeht. Ein Füllvolumen von 2 ml entspricht bei Ausführungs- und Vergleichsbeispiel dem Nennvolumen. Somit bedeckt der größte Teil der Flüssigkeit die unverformten Wandbereiche des Glasfläschchens. Entsprechend wird der Einfluss der hohen Migrationslast durch bodennahe Bereiche sehr gering bis vernachlässigbar.

Die Konzentrationsverhältnisse der ausgelösten Bestandteile bei einem Füllvolumen von 0,5 ml und 2 ml bzw. bei einem Füllvolumen von 1 ml und 2 ml sind dabei ein Maß für die Abweichung der Auslösestärke, die eine Flüssigkeit bei sehr geringen bzw. geringen Füllgraden erfährt zur Auslösestärke bei einem hohen Füllungsgrad. Ein Verhältnis von 1,6 würde dabei bedeuten, dass es keinen Unterschied in der Auslösestärke des entsprechenden Füllungsgrad zur Auslösestärke bei einem Füllungsgrad, der dem Nennvolumen des Glasfläschchens entspricht, gibt.

Das Verhältnis der Konzentrationen bei 1 ml Füllvolumen und 2 ml Füllvolumen lässt dabei Rückschlüsse zu, bis zu welcher Höhe, d.h. bis zu welcher Entfernung zum Boden das Glasfläschchen, die Innenwandung des Glasfläschchens bedingt durch den Herstellungsprozess, eine erhöhte Auslösestärke aufweist.

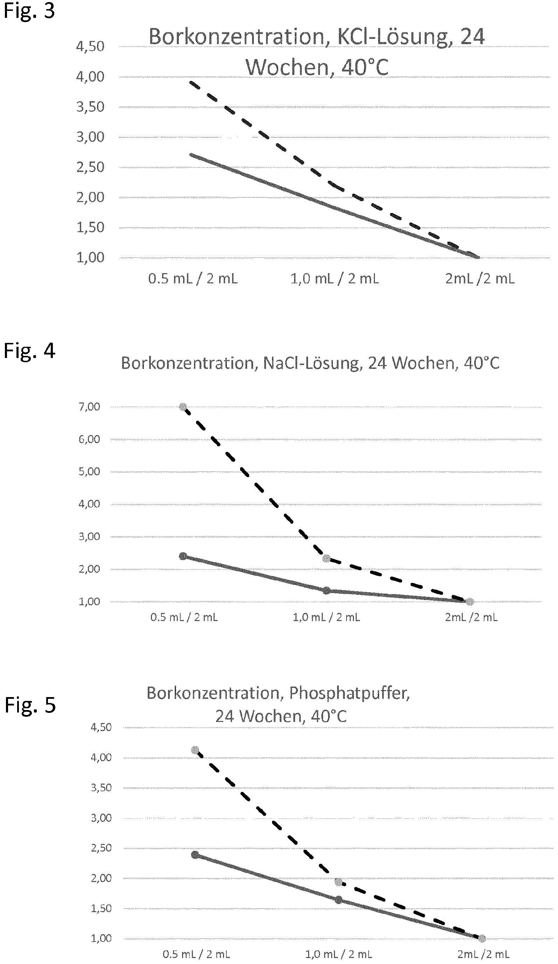

Aus den Fig. 2 bis 5 wird dabei ersichtlich, dass die Migrationslast, die durch Abgabe von Borionen aus den vom bodennahen Wandbereich ausgeht beim Ausführungsbeispiel (durchgezogene Linie) wesentlich geringer ist als beim Vergleichsbeispiel (gestrichelte Linie). Dies trifft hierbei für alle vier Auslösungsmedien zu. Beispielsweise ist das Konzentrationsverhältnis von 0,5 ml Füllvolumen zu 2 ml Füllvolumen bei NaCl-Lösung als Auslösungsmedium (Fig. 4) beim Ausführungsbeispiel um mehr als den Faktor 3 geringer als beim Vergleichsbeispiel, d.h. die Auslösestärke der bodennahen Wandbereiche ist beim Ausführungsbeispiel wesentlich geringer als bei dem Vergleichsbeispiel. Darüber hinaus ist bei allen Auslösemedien beim Ausführungsbeispiel das Konzentrationsverhältnis zwischen 1 ml und 2 ml wesentlich geringer als beim Vergleichsbeispiel.

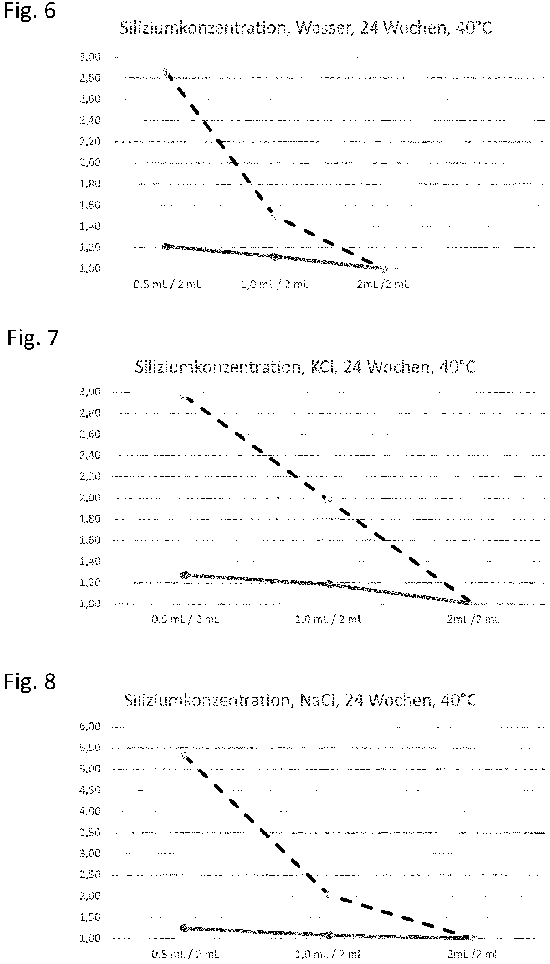

Ein ähnliches Auslöseverhalten ist auch für die Glasbestandteile Silizium, Natrium und Calcium zu beobachten. So wird auch aus den Fig. 6 bis 16 deutlich, dass die Migrationslast, die durch Abgabe der Glasbestandteile aus dem bodennahen Wandbereich ausgeht, beim Ausführungsbeispiel (durchgezogene Linie) wesentlich geringer ist als beim Vergleichsbeispiel (gestrichelte Linie). Dies wird beispielsweise an Hand des Auslösungsverhaltens von Silizium in den unterschiedlichen Auslösemedien deutlich (Fig. 6 bis 10). So zeigt Fig. 8, dass beim Ausführungsbeispiel das Konzentrationsverhältnis von 0,5 ml zu 2 ml sogar kleiner als 1,5 ist, während das Vergleichsbeispiel ein Verhältnis von mehr als 5 zeigt. Ein Konzentrationsverhältnis von 0,5 ml zu 2 ml von kleiner 1,6 bedeute für die verwendeten Glasfläschchen, dass die Auslösestärke bei einem Füllvolumen von 0,5 ml sogar kleiner ist als die Auslösestärke bei einem Füllvolumen von 2 ml.

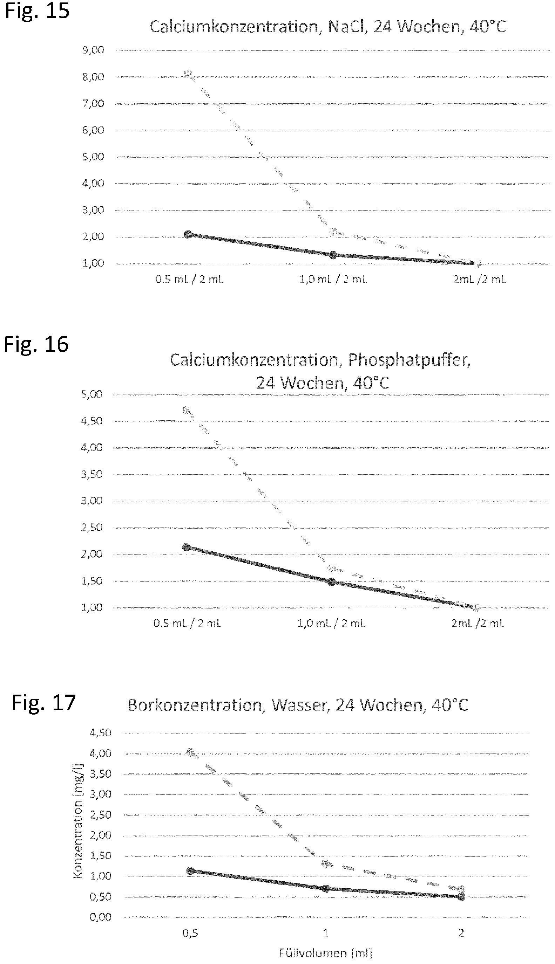

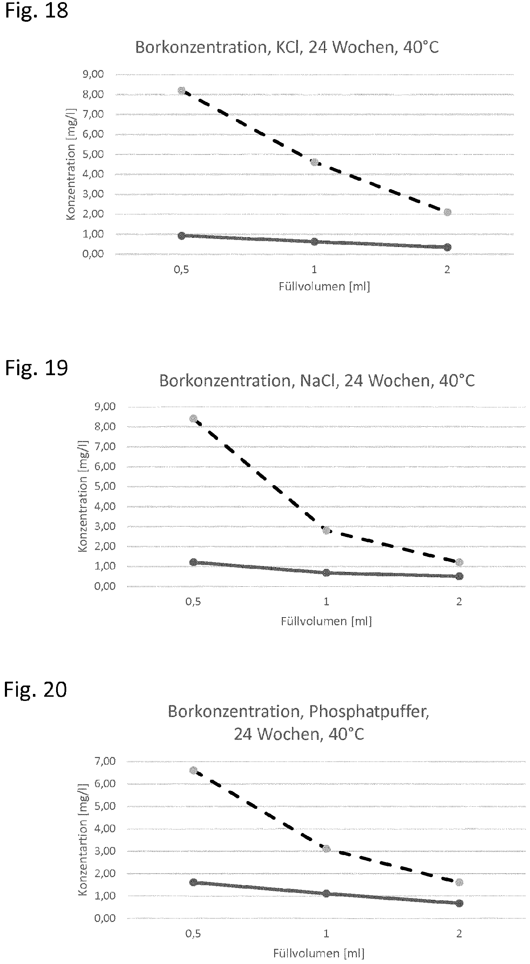

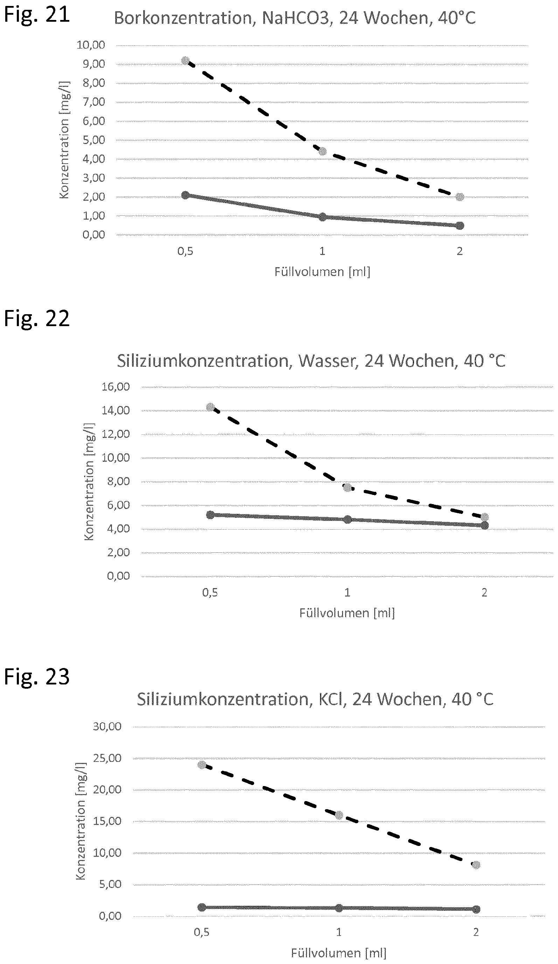

In den Fig. 17 bis 31 werden die ermittelten Konzentrationen der ausgelösten Glasbestandteile in verschiedenen Auslösemedien von Ausführungsbeispiel und Vergleichsbeispiel dargestellt. Der größte Unterscheid zwischen Ausführungsbeispiel und Vergleichsbeispiel kann bei mit KCl gefüllten Glasfläschchen mit einem Füllvolumen von 0,5 ml beobachtet werden (Fig. 18, 23, 28, 30). Fig. 23 zeigt hierbei, dass sich die Siliziumkonzentrationen um einen Faktor von etwa 16 unterscheiden.

Nach einer Lagerung bei 40°C für t2 = 48 Wochen zeigte das Ausführungsbeispiel eine überlegene chemische Beständigkeit gegenüber dem Vergleichsbeispiel, insbesondere, wenn die Glasfläschchen mit einem niedrigen Füllvolumen gefüllt waren. Dieser Effekt wurde bei allen als Füllung verwendeten Lösungen beobachtet: aufbereitetes Wasser, 0,9% NaCl, phosphathaltige Pufferlösung, 8,4% NaHCO₃ und 15% KCl sowie bei Glasfläschchen mit und ohne Dampfsterilisation.

Bei allen dargestellten Auslösemedien und ausgelösten Bestandteilen gilt, dass beim Vergleichsbeispiel sowohl (wie oben beschrieben und in den Fig. 2 bis 16 dargestellt) das Konzentrationsverhältnis von 0,5 ml zu 2 ml größer ist als beim Ausführungsbeispiel, wie auch die gemessenen Konzentrationen der jeweiligen ausgelösten Glasbestandteile. Dies bedeutet, dass beim Ausführungsbeispiel insgesamt die Migrationslast kleiner ist als beim Vergleichsbeispiel. Dieser Unterschied beschränkt sich hierbei nicht nur auf die bodennahen Wandbereiche, sondern ist in oberen Wandbereichen, die durch das Füllvolumen von 2 ml repräsentiert werden, gegeben.

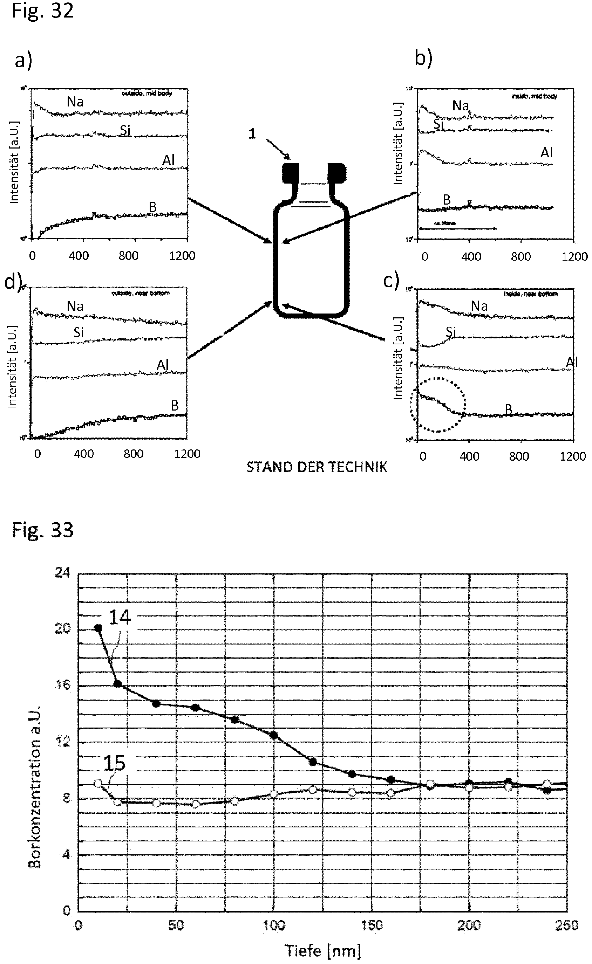

In Fig. 32 werden die an einem Vergleichsbeispiel mittels SIMS ermittelten Tiefenprofile der Glasbestandteile Bor, Natrium, Aluminium und Silizium dargestellt. Hierbei wurden Tiefenprofile an vier verschiedenen Stellen des Glasfläschchens gemessen, nämlich auf der Außenwandung in einem unverformten, oberen Wandbereich des Fläschchens (a)), auf der Innenwandung in einem unverformten, oberen Wandbereich des Fläschchens (b)), auf der Innenwandung in einem bodennahen Wandbereich (c)) und auf der Außenwandung in einem bodennahen Wandbereich. Die in der x-Achse des Diagramms aufgetragenen Sputterzeiten sind dabei ein Maß für die jeweilige Glastiefe. So können hohen Sputterzeiten tiefe Bereiche im Glas zugeordnet werden.

Bei den Profilen b) aus den unverformten Wandbereichen ist bei geringen Sputterzeiten und somit in den oberflächennahen Glasbereichen das Natriumsignal stark erhöht, während bei den übrigen Glasbestandteilen im Wesentlichen keine Überhöhung zu beobachten ist. Es kann daraus geschlossen werden, dass in den unverformten Wandbereichen vorwiegend Natriumionen abgegeben werden. Somit wird in diesen Bereichen der größte Teil der Migrationslast durch die abgegebenen Natriumionen gebildet.

Die Tiefenprofile c) aus den bodennahen Wandbereichen zeigen neben einer Erhöhung der Natriumionensignale in oberflächennahen Glasbereichen eine deutliche Überhöhung der Borsignale in diesen Bereichen. Eine starke Überhöhung der Borsignale in oberflächennahen Bereichen führt hierbei nicht nur zu einer erhöhten Migrationslast durch ausgelöste Borionen, sondern es kann durch die Konzentrationserhöhung auch zu einer oberflächennahen Phasenseparation des Glases kommen, was zu einer Verringerten chemischen Stabilität führen kann.

In Fig. 33 sind die Konzentrationstiefenverläufe von Bor in einem bodennahen Wandbereich eines Ausführungsbeispiels (Kurve 2) sowie eines Vergleichsbeispiels (Kurve 1) dargestellt. Es wird deutlich, dass die Überhöhung der Borionenkonzentration beim Ausführungsbeispiel wesentlich geringer ist als beim Vergleichsbeispiel. Beide Gläser zeigen dabei ab einer Tiefe von ca. 200 nm einen vergleichbaren Plateauwert für die Borionenkonzentration.

Während das Ausführungsbeispiel jedoch bei einer Tiefe im Bereich von 10 bis 30 nm eine Überhöhung der Borkonzentration von weniger als 15 % gegenüber dem Plateauwert für die Borkonzentration ab einer Tiefe von 150 nm zeigt, so weist das Vergleichsbeispiel eine entsprechende Konzentrationsüberhöhung von mehr als 100% auf. Zudem sinkt die Borkonzentration weniger stark als beim Ausführungsbeispiel, so dass der Plateauwert für die Borionenkonzentration erst bei einer Tiefe von ca. 150 nm erreicht wird, während beim Ausführungsbeispiel der Plateauwert schon bei einer Tiefe von 100 nm erreicht wird.

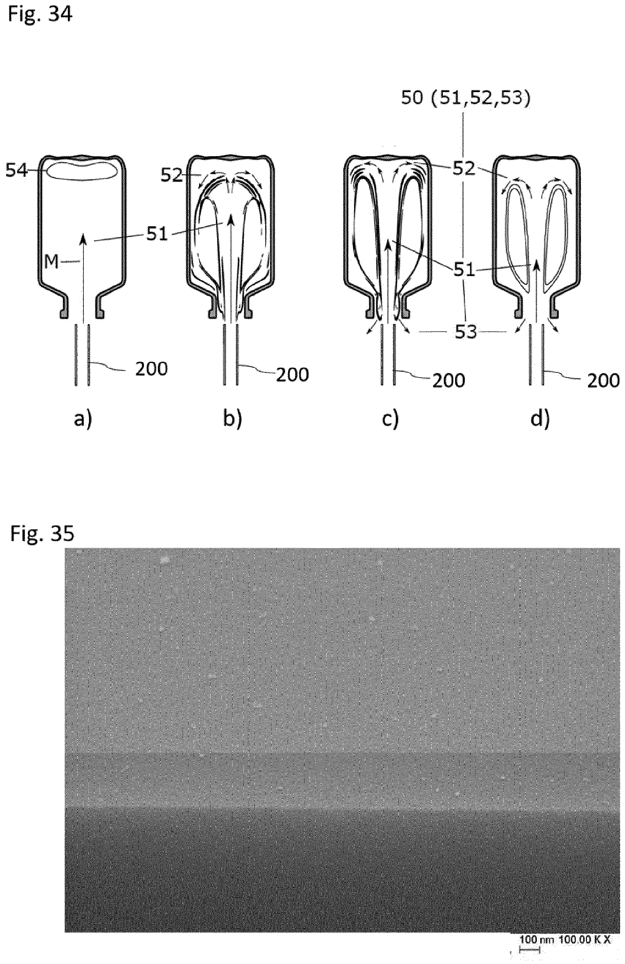

Details zu einem möglichen Herstellungsverfahren der erfindungsgemäßen Glasfläschchen sind in den Figuren 34a bis 34d gezeigt. Das Verfahren umfasst dabei zumindest die folgenden Schritte
- Lokales lokales Erwärmen eines Endes eines Glasrohrs
- das Abtrennen des lokal erwärmten Endes des Glasrohrs unter Ausbildung des Glasfläschchens mit einem geschlossenen Boden, und
- weitere Formung des Bodens des Glasfläschchens.

Das ausgebildete Glasfläschchen wird dabei vorzugsweise nach dem Abtrennen von dem Glasrohr kopfüber gehalten und während der weiteren Formung des Bodens mit Hilfe eines Spülgases durchgespült, so dass eine Spülgasströmung im Inneren des Glasfläschchens erzeugt wird. Es hat sich als besonders vorteilhaft herausgestellt, wenn das Spülgas über die Einfüllöffnung mittig ein- oder ausströmt und außermittig aus- oder einströmt.

In den Figuren 34a-34d sind vier Phasen eines Spülvorgangs einer Ausführungsform des oben beschriebenen Herstellungsverfahrens dargestellt. Im Folgenden werden die einzelnen Phasen während der weiteren Formung der Böden der Glasfläschchen beschrieben:
- erste Phase (vgl. Fig. 34a): Start des Spül-Prozesses, wobei in dieser Phase zunächst eine Spülgasströmung 50 im Inneren des Glasbehälters aufgebaut wird, und wobei das dabei aus dem Rohr 200 ausströmende Spülgas mit einem angemessenen Druck in das Innere des Glasfläschchens 100 geblasen wird, so dass dieser eintretende Spülgasströmungs-Anteil 51 zunächst gegen das heiße Gas 54 an der Bodenzone des Glasbehälters drückt
- zweite Phase (vgl. Fig. 34b): Ausbilden eines reinigenden Spülgasströmungs-Anteils 52, wobei sich dieser reinigende Spülgasströmungs-Anteil 52 zwischen dem heißen Gas 54 an der Bodenzone des Glasbehälters und dem eintretenden Spülgasströmungs-Anteil 51 in der Nähe des Glasfläschchen-Bodens halbkreisförmig ausbildet. Diese Phase beginnt unmittelbar nach der ersten Phase, was insbesondere von dem Druck des einströmenden Spülgases und den geometrischen Verhältnissen in der Umgebung des vorderen Endes des Rohrs und der Einfüllöffnung abhängt.
- dritte Phase (vgl. Fig. 34c): Ausbilden eines austretenden Spülgasströmungs-Anteils 53, wobei dieser austretende Spülgasströmungs-Anteil 53 allenfalls minimal mit dem eintretenden Spülgasströmungs-Anteil 51 und dem reinigenden Spülgasströmungs-Anteil 52 wechselwirkt und insbesondere keine Turbulenzen ausbildet, so dass das verunreinigte, heiße Spülgas 54 aus dem Glasfläschchen herausgeblasen oder abgesaugt wird.
- vierte Phase (vgl. Fig. 34d): Beenden des Spül-Prozesses, wobei der Druck des einströmenden Spülgases 50 vermindert wird und die letzten Verunreinigungen aus dem Glasfläschchen heraus gespült werden.

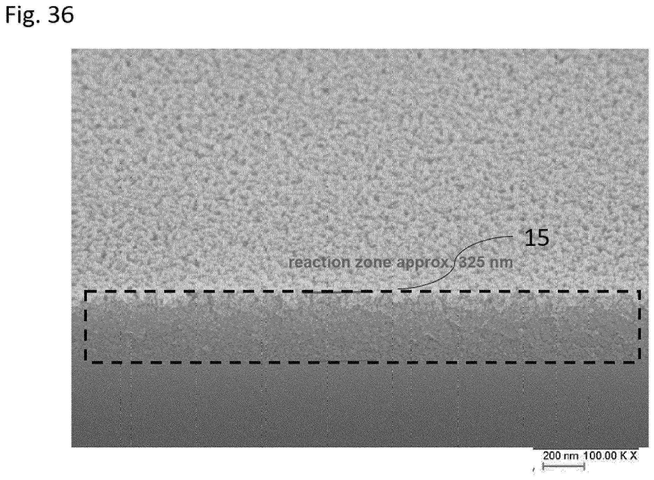

Die Figuren 35 und 36 zeigen SEM Querschnittsanalysen in der Wandung im bodennahen Wandbereich für jeweils ein ausgewähltes Ausführungsbeispiel (Fig. 35) und ein Vergleichsbeispiel (Fig. 36). Beide Gläschen wurden zuvor über 48 Wochen mit 0,5 ml einer NaCl-Lösung gefüllt und bei 40°C stehend gelagert.

Fig. 35 zeigt hierbei die homogene Struktur des Glases beim Ausführungsbeispiel. Größere Oberflächendefekte sind nicht erkennbar, das Glas ist im untersuchten Querschnittsbereich ohne strukturelle Auffälligkeiten. Im Unterschied dazu weist das Glas des Vergleichsbeispiels eine poröse Schicht auf, die sich bis zu einer Tiefe von ca. 325 nm erstreckt (Reaktionsschicht 16). Diese Schicht ist durch die Wechselwirkung der NaCl-Lösung mit der Phasen-separierten Glasschicht entstanden. Die dadurch entstandene Mikrorauheit kann dabei auch als Anzeichen für eine Glaskorrosion gewertet werden. Beim Vergleichsbeispiel wird der bodennahe Wandbereich durch die Phasentrennung verändert...

Beim Ausführungsbeispiel tritt eine derartige Phasenseparation dagegen nicht auf, was zu der hohen chemischen Beständigkeit und vergleichsweise geringen Migrationslast selbst in den bodennahen Wandbereichen führt.

Die hohe chemische Beständigkeit kann auch mit Hilfe des sogenannten "Quick-Tests", d.h. eines Schnelltests verdeutlicht werden. Allgemein gibt der Quicktest dabei Aufschluss, in wie weit eine Reaktionsschicht von der Innenwand eines Glasfläschchens ausgebildet ist. Eine Reaktionsschicht führt dabei im Allgemeinen zu einer geringeren chemischen Beständigkeit sowie zu einer erhöhten Tendenz zur Schichtablösung. Ein Indikator für das Risiko der Schichtablösung ist hierbei die aus dem Glasfläschchen unter den standardisierten Testbedingungen ausgelöste Menge an Natriumoxid.

Hierbei wurden Glasfläschchen mit verschiedenen Nennvolumina mit zwei verschiedenen Verfahren zur Dampfsterilisation belastet und die Natriumabgabe der Innenfläche gemessen.

Hierbei erfolgte der Test unter Berücksichtigung der Normen "European Pharmacopeia, Kapitel 3.2.1 sowie der ISO 4802-2.

Die leeren Glasfläschchen wurden mit dem Boden nach oben durch Dampfsterilisation belastet, so dass es für 240 Minuten bei 121°C zu einer Wechselwirkung mit der inneren Atmosphäre kommt. Anschließend werden die Glasfläschchen, abhängig vom Nominalvolumen, mit dem gegebenen Füllvolumen gefüllt und wiederum mit Dampf sterilisiert. Letzteres geschieht für 120 Minuten bei 121°C. Die Abgabe von Natrium wird mittels Flammenatom Absorptionsspektroskopie gemäß der ISO 4802-2 gemessen.

Die folgenden Reagenzien wurden verwendet:
- Wasser P: aufbereitetes Wasser mit einer Leitfähigkeit < 5 µS/cm) bei 25°C
- Testwasser P1: Frisch aufbereitetes Wasser mit einer Leitfähigkeit < 1 µS/cm bei 25°C
- Caesiumchlorid (CsCl):suprapur Merck, Nr. 1.02039.0250
- Salzsäure, suprapur c(HCl) 0 30%, Merck Nr. 1.00318.1000
- Salzsäure, c(HCl) 0 6 mol/L, 635 ml der Salzsäure c(HCl) = 30% müssen mit dem Testwasser P1 auf 1000 ml verdünnt werden. Haltbarkeit: 12 Monate
- Spektrochemische Pufferlösung: Lösen von 80 g CsCl in etwa 500 ml Testwasser P1, Hinzugabe von 10 ml Salzsäure suprapur und Auffüllen mit Testwasser P1 auf 1000 ml. Haltbarkeit: 12 Monate
- Natriumstandardlösung, c(Na) = 1000 mg/l, Merck Nr. 1703563 (gebrauchsfertig), alternativ Merck Nr. 1.09927.0001 (Titrisol Ampulle, Verwendung wie auf der Packung beschrieben, gewaschen und mit Testwasser P! auf 1000 ml aufgefüllt. Haltbarkeit: 12 Monate
- Natriumstammlösung, c(Na) = 100 mg/l, 100 ml der Natriumstammlösung müssen mit Testwasser P1 auf 1000 ml verdünnt werden. Haltbarkeit: 3 Monate
- Kalibrierungsstandards für die Natriummessungen: In jedem 100 ml Messkolben aus Plastik müssen die folgenden Volumina der Natriumstammlösung und der spektrochemischen Pufferlösung unter Verwendung einer Pipette umgefüllt und mit Testwasser P1 auf 100 ml aufgefüllt werden.

**Tabelle 20: Kalibrierungsstandards**

| | Standard 1 | Standard 2 | Standard 3 | Standard 4 | Standard 5 |
|---|---|---|---|---|---|
| Konzentration | 0 mg/l | 1 mg/l | 2 mg/l | 4 mg/l | 5 mg/l |
| Volumen Stammlösung | 0 ml | 1 ml | 2 ml | 4 ml | 5 ml |
| Spektrochemische Pufferlösung | 5 ml | 5 ml | 5 ml | 5 ml | 5 ml |

Das Füllvolumen hängt vom Rohrdurchmesser ab, der für die jeweiligen Glasfläschchen verwendet wurde.

**Tabelle 11: Füllvolumen für Behälter, die aus Röhren mit verschiedenen Durchmessern hergestellt wurden**

| Außen-Durchmesser Behälterkörper QJ mm | Quick Test Füllvolumen ml | Artikel oder Nominalvolumen | Anzahl der Vials, die für die Messung zusammen genommen wurden |
|---|---|---|---|
| 16,00 | 1,00 | 2R | 2 |
| 20,50 | 3,00 | | 1 |
| 22,00 | 3,50 | 6 ( 8 R | 1 |

Vor dem Test wird jeder Behälter randvoll mit Wasser P oder P1 bei 50°C (± 5 °C) gefüllt und für 20 Minuten stehen gelassen. Anschließend erfolgt ein Leeren der Behälter und dreimaliges Spülen jeden Behälters mit Wasser P1 von Raumtemperatur 20°C (± 5 °C). Zu beachten ist, dass die Wassertemperaturen mit einem Temperaturmessgerät zu überprüfen sind.

In einer ersten Phase werden die leeren Behälter durch Dampfsterilisation belastet: Unmittelbar nach der Reinigung werden die Glasfläschchen so im Dampfsterilisators angeordnet, so dass die Behälter mit dem Boden nach oben stehen, um einen permanenten Austausch mit der Atmosphäre des Dampfsterilisators zu erlauben. Das Thermoelement des Dampfsterilisators wird in der Luft des Kessels angeordnet. Bei Dampfsterilisatoren gemäß ISO 4802-2 wird der Dampfsterilisator auf 100°C erhitzt. Der Dampf soll für 10 Minuten aus dem Entlüftungshahn entweichen. Dann wird der Entlüftungshahn geschlossen und die Temperatur mit einer Rate von 1°C pro Minute von 100°C auf 121 °C erhöht: Anschließend wird die Temperatur für 240 ± 1 Minuten bei 121 ± 1°C gehalten. Dann wird die Temperatur mit einer Rate von 0,5°C pro Minute von 121°C auf 100°C gesenkt, und belüftet. Anschließend werden die Behälter bei den normalen Vorsichtsmaßnahmen aus dem Dampfsterilisator entfemt und nur in Luft abgekühlt.

Die Gläschen werden dann gemäße Tabelle 21 mit Wasser P1 gefüllt. Jeder einzelne Behälter soll locker mit einem Stück Aluminiumfolie verschlossen werden, die zuvor mit Wasser P1 gespült wurde. Bei Dampfsterilisatoren gemäß ISO 4802-2 wird der Dampfsterilisator auf 100°C erhitzt und der Dampf soll für 10 Minuten aus dem Entlüftungshahn entweichen. Dann wird der Entlüftungshahn geschlossen und die Temperatur mit einer Rate von 1°C pro Minute von 100°C auf 121 °C erhöht: Anschließend wird die Temperatur für 120 ± 1 Minuten bei 121 ± 1°C gehalten. Dann wird die Temperatur mit einer Rate von 0,5°C pro Minute von 121°C auf 100°C gesenkt, hierbei ist zu lüften um die Entstehung eines Vakuums zu verhindern. Der Dampfsterilisator darf nicht geöffnet werden, bevor er auf 95°C abgekühlt ist.

Das Kühlen der Gläschen erfolgt durch Luftventilation und die Proben werden für die FAAS-Messung vorbereitet. Hierzu wird jedem Gläschen ein Volumen der spektrochemischen Pufferlösung entsprechend 5% des Füllvolumens zugegeben und anschließend durch Rühren in der Lösung im Glasfläschchen vermischt. Anschließend werden die Glasfläschchen vollständig entleert und die Extraktionslösungen werden in eine Kunststoffröhre gefüllt und mit der spektrochemischen Pufferlösung versetzt. Das Volumen der zugegebenen spektrochemischen Pufferlösung entspricht 5% des zusammengegebenen Gesamtvolumens.

Die Bestimmung der Natriumabgabe mittels FAAS erfolgt wie folgt:
Die Extraktionslösung wird aus der Plastikröhre direkt in die Flamme des Atomabsorptionsinstruments angesaugt und die genäherte Konzentration von Natriumoxid wird durch Bezugnahme auf den Klalibriergraphen bestimmt, der mittels der Referenzlösungen geeigneter Konzentration bestimmt wurde. Anschließend wird der Mittelwert der in jeder getesteten Probe gefundenen Natriumkonzentration berechnet, in Mikrogramm an Natriumoxid Na₂O pro Milliliter der Extraktion.
Na₂O [mg/l] = Na [mg/l] x 1,348 x 1,05
(Faktor Na - Na₂O = 1,348; Verdünnungsfaktor = 1,05)

Die erfindungsgemäßen Glasfläschchen weisen dabei abhängig von ihrem Volumen Natriumoxidkonzentrationen innerhalb der Grenzwerte gemäß Tabelle 23 auf.

**Tabelle 22: Grenzwerte für den durch den Behältertypen definierten Na₂O Gehalt.**

| Behälterkörper QJ mm | Füllvolumen ml | Artikel- oder Nominalvolumen | Grenzwert für Quicktest Na2O Gehalt Mg/l |
|---|---|---|---|
| 16,00 | 1,00 | 2R | 4,5 ± 0,3 |
| 22,00 | 3,50 | 10 R | 2,7 ± 0,2 |
| 24,00 | 4,00 | 50 R | 2,5 ± 0,2 |

Bevorzugt ist die durch den oben beschriebenen Quicktest ermittelte Natriumoxidkonzentration bei einem Glasfläschchen mit einem Nennvolumen von 2 ml kleiner als 5 mg/l. Die Natriumoxidkonzentration ist hierbei ein Maß für das Risiko der Ablösung einer Reaktionsschicht von der Innenwand des Glasfläschchens und zeigen die hohe chemische Stabilität des erfindungsgemäßen Glasfläschchens.

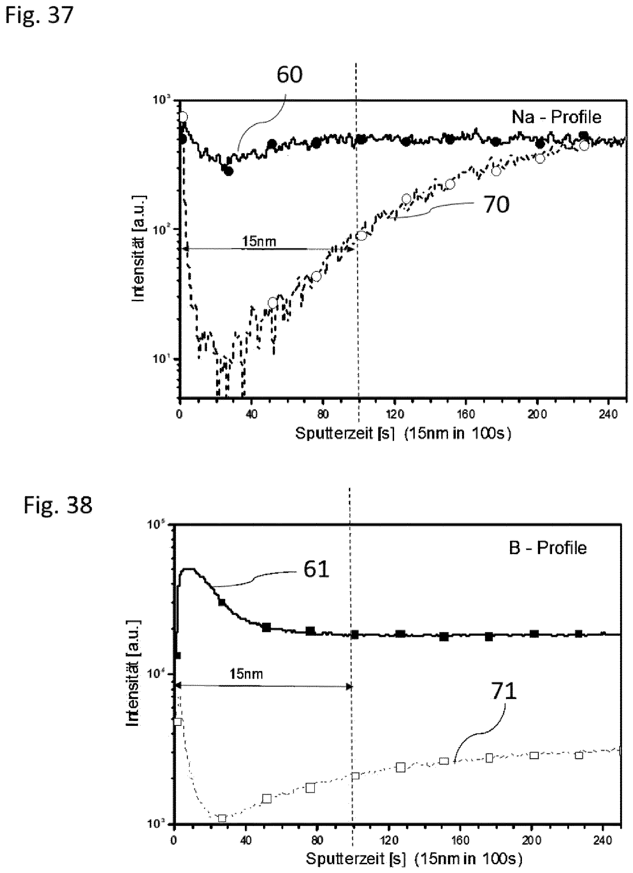

In Fig. 37 sind die Tiefenverläufe von Natriumsignalen aus dem oberen Wandbereich (60) und aus dem Boden (70) eines Glasfläschchens bis zu einer Tiefe von ca. 35 nm dargestellt. Es wird deutlich, dass eine starke Verarmung von Natrium in der oberflächennahen Glasschicht im Boden vorliegt. Fig. 38 zeigt die Tiefenverläufe von Borsignalen aus dem oberen Wandbereich (61) und aus dem Boden (71) eines Glasfläschchens bis zu einer Tiefe von ca. 35 nm. Hier ist eine sehr starke Verarmung von Bor in der oberflächennahen Glasschicht gegenüber einer vergleichbaren Glasschicht eines oberen Wandbereichs zu erkennen.

## Patentansprüche

1. Verwendung eines Glasfläschchens (1) mit einem Gesamtvolumen < 4,5 ml aus einem Mehrkomponentenglas, wobei die Innenwand (21) des Glasfläschchens (1) eine chemische Beständigkeit gegen ein Auslösen zumindest eines der Bestandteile des Mehrkomponentenglases aufweist, wobei
bei Auslösen des Glasfläschchens (1) mit einer wässrigen Flüssigkeit mit einem pH-Wert im Bereich von 5 bis 9 bei einer Temperatur von 40°C über einen Zeitraum von 24 Wochen bei stehender Lagerung des Glasfläschchens das Verhältnis der Konzentration zumindest eines ausgelaugten Bestandteiles bei einem Füllvolumen von 0,5 ml und der Konzentration bei einem Füllvolumen von 2 ml maximal 3 und das Verhältnis zwischen der Konzentration bei einem Füllvolumen von 1 ml und der Konzentration bei einem Füllvolumen von 2 ml maximal 2 liegt,
zur Befüllung mit einer flüssigen pharmazeutischen Zubereitung (4) bis zu einem Befüllungsgrad von maximal 0,25.

2. Verwendung eines Glasfläschchens (1) gemäß Anspruch 1, wobei bei Auslösen des Glasfläschchens (1) mit einer wässrigen Flüssigkeit mit einem pH-Wert im Bereich von 5 bis 9 bei einer Temperatur von 40°C über einen Zeitraum von 24 Wochen bei stehender Lagerung des Glasfläschchens das Verhältnis der Konzentration des ausgelösten Bestandteiles bei einem Füllvolumen von 0,5 ml und der Konzentration bei einem Füllvolumen von 2 ml maximal 2,5, besonders bevorzugt maximal 1,5 und/oder das Verhältnis zwischen der Konzentration bei einem Füllvolumen von 1 ml und der Konzentration bei einem Füllvolumen von 2 ml maximal 1,5 und/oder das Verhältnis zwischen der Konzentration des ausgelaugten Bestandteils bei einem Füllvolumen von 0,5 ml und der Konzentration bei einem Füllvolumen von 1 ml maximal 2,5, bevorzugt maximal 1,7 beträgt.

3. Verwendung eines Glasfläschchens (1) gemäß Anspruch 1, wobei als Flüssigkeit zum Auslösen der Glasbestandteile aufbereitetes Wasser, 10%ige KCI-Lösung, 0,5%ige NaCl-Lösung, ein Phosphatpuffer oder eine NaHCOs-Lösung, bevorzugt aufbereitetes Wasser verwendet wird.

4. Verwendung eines Glasfläschchens (1) gemäß einem der vorstehenden Ansprüche, wobei das Glasfläschchen (1) aus einem Mehrkomponentenglas umfassend zumindest Si und Na, bevorzugt umfassend Si und Na und zumindest eines der Bestandteile aus der Gruppe gebildet von B, Al, und/oder Ca, besteht und/oder das Mehrkomponentenglas ein Borosilikatglas, bevorzugt ein Neutralglas der Klasse I, ist.

5. Verwendung eines Glasfläschchens (1) gemäß einem der vorstehenden Ansprüche, wobei bei Auslösen des Glasfläschchens (1) mit aufbereitetem Wasser bei einer Temperatur von 40°C und einer Lagerdauer von 24 Wochen bei aufrechter Lagerung des Glasfläschchens das Verhältnis zwischen der Konzentration des ausgelösten Bestandteils bei einem Füllvolumen von 1 ml und der Konzentration des ausgelaugten Bestandteils bei einem Füllvolumen von 2 ml für Silizium maximal 1,5, für Natrium maximal 1,6 und/oder für Bor maximal 2 beträgt.

6. Verwendung eines Glasfläschchens (1) gemäß der vorstehenden Ansprüche wobei bei Auslösen des Glasfläschchens (1) mit einer wässrigen Flüssigkeit mit einem pH-Wert im Bereich von 5 bis 9 die Auslösestärke für den ausgelösten Bestandteil bei einem Füllvolumen von 0,5 ml kleiner ist als die Auslösestärke für den ausgelösten Bestandteil bei einem Füllvolumen von 2 ml.

7. Verwendung eines Glasfläschchens (1) gemäß einem der vorstehenden Ansprüche, wobei bei Auslösen des Glasfläschchens (1) mit 15%iger KCI-Lösung bei einer Temperatur von 40°C und einer Lagerdauer von 24 Wochen bei aufrechter Lagerung des Glasfläschchens bei einem Füllvolumen von 0,5 ml die Konzentration des ausgelaugten Bestandteiles für Silizium im Bereich von 1 bis 3 mg/l, für Bor im Bereich von 0,2 bis 1,2 mg/l, für Natrium im Bereich von 1,8 bis 3,5 mg/l und/oder für Calcium im Bereich von 0,2 bis 1 mg/l und/oder wobei bei Auslösen des Glasfläschchens (1) mit 0,9%iger NaCl-Lösung Wasser bei einer Temperatur von 40°C und einer Lagerdauer von 24 Wochen bei aufrechter Lagerung des Glasfläschchens bei einem Füllvolumen von 0,5 ml die Konzentration des ausgelösten Bestandteiles für Silizium im Bereich von 2 bis 4 mg/l, für Bor im Bereich von 0,6 bis 1,5 mg/l und/oder für Calcium im Bereich von 0,2 bis 1 mg/l liegt.

8. Verwendung eines Glasfläschchens (1) gemäß einem der vorstehenden Ansprüche wobei bei Auslösen des Glasfläschchens (1) mit 0,9%iger NaCl-Lösung bei einer Temperatur von 40°C und einer Lagerdauer von 24 Wochen bei aufrechter Lagerung des Glasfläschchens das Verhältnis zwischen der Konzentration des ausgelösten Bestandteils bei einem Füllvolumen von 0,5 ml und der Konzentration des ausgelösten Bestandteils bei einem Füllvolumen von 2 ml für maximal 2,5, bevorzugt maximal 1,5 beträgt und/oder das Verhältnis zwischen der Konzentration des ausgelösten Bestandteils bei einem Füllvolumen von 1 ml und der Konzentration des ausgelösten Bestandteils bei einem Füllvolumen von 2 ml für Silizium maximal 1,6, bevorzugt maximal 1,5 beträgt.

9. Verwendung eines Glasfläschchens (1) gemäß einem der vorstehenden Ansprüche, wobei die flüssige pharmazeutische Wirkstoffzubereitung (4) therapeutische Proteine, monoklonale Antikörper und/oder Vakzine enthält.

10. Verwendung eines Glasfläschchens (1) gemäß einem der vorstehenden Ansprüche, wobei das Glasfläschchen (1) mit einem Verfahren umfassend zumindest die folgenden Schritte herstellbar ist:
- lokales Erwärmen eines Endes eines Glasrohrs,
- Abtrennen des lokal erwärmten Endes des Glasrohrs unter Ausbildung eines Glasfläschchens mit einem geschlossenen Boden, und
- weitere Formung des Bodens (3) des Glasfläschchens (1), wobei:
- das ausgebildete Glasfläschchen (1); und
- bei der weiteren Formung des Bodens des Glasfläschchens mit Hilfe eines Spülgases, eine Spülgasströmung (50) im Inneren der Glasfläschchen erzeugt wird.

11. Glasfläschchen (1) aus einem borhaltigen Mehrkomponentenglas mit einer flüssigen wässrigen pharmazeutischen Wirkstoffzubereitung (4) mit einem pH-Wert im Bereich von 5 bis 9 und einem Sterilverschluss, wobei das Glasfläschchen (1) ein Gesamtvolumen < 4,5 ml aufweist, der Befüllungsgrad des Glasfläschchens (1) mit der pharmazeutische Wirkstoffzubereitung (4) maximal 0,25 beträgt und wobei die Innenwand (21) des Glasfläschchens (1) eine chemische Beständigkeit gegen ein Auslösen zumindest eines der Bestandteile des Mehrkomponentenglases aufweist, wobei das Verhältnis der Konzentration zumindest eines ausgelösten Bestandteiles bei einem Füllvolumen von 0,5 ml und der Konzentration bei einem Füllvolumen von 2 ml maximal 3 und das Verhältnis zwischen der Konzentration bei einem Füllvolumen von 1 ml und der Konzentration bei einem Füllvolumen von 2 ml maximal 2 beträgt.

12. Glasfläschchen (1) gemäß Anspruch 11, wobei bei Auslösen des Glasfläschchens (1) mit einer Flüssigkeit das Verhältnis zwischen der Konzentration des ausgelaugten Bestandteils bei einem Füllvolumen von 0,5 ml und der Konzentration bei einem Füllvolumen von 1 ml maximal 2,5, bevorzugt maximal 1,7 beträgt.

13. Glasfläschchen (1) gemäß einem der vorstehenden Ansprüche 11 oder 12, wobei das Glasfläschchen (1) aus einem Mehrkomponentenglas umfassend Si und Na, bevorzugt umfassend Si, Na und zumindest eines der Bestandteile aus der Gruppe gebildet von B, AI und/oder Ca, bevorzugt aus einem Borosilikatglas und besonders bevorzugt aus einem Neutralglas besteht.

14. Glasfläschchen (1) gemäß einem der vorstehenden Ansprüche 11 bis 13, wobei bei Auslösen des Glasfläschchens (1) mit Wasser, bevorzugt mit aufbereitetem Wasser, bei einer Temperatur von 40°C und einer Lagerdauer von 24 Wochen bei aufrechter Lagerung des Glasfläschchens das Verhältnis zwischen der Konzentration des ausgelösten Bestandteils bei einem Füllvolumen von 0,5 ml und der Konzentration des ausgelösten Bestandteils bei einem Füllvolumen von 1 ml für Silizium maximal 1,5, für Natrium maximal 2,5 und/oder für Bor maximal 3 und/oder das Verhältnis zwischen der Konzentration des ausgelösten Bestandteils bei einem Füllvolumen von 1 ml und der Konzentration des ausgelösten Bestandteils bei einem Füllvolumen von 2 ml für Silizium maximal 1,5, für Natrium maximal 1,6 und/oder für Bor maximal 2 beträgt.

15. Glasfläschchen (1) gemäß einem der vorstehenden Ansprüche 11 bis 14, wobei bei Auslösen des Glasfläschchens (1) mit Wasser bei einer Temperatur von 40°C und einer Lagerdauer von 24 Wochen bei aufrechter Lagerung des Glasfläschchens bei einem Füllvolumen von 0,5 ml die Konzentration des ausgelösten Bestandteiles für Silizium im Bereich von 3 bis 6 mg/l, für Bor im Bereich von 0,8 bis 1,6 mg/l, für Natrium im Bereich von 1,6 bis 4 mg/l, für Calcium im Bereich von 0,05 bis 0,5 mg/l und/oder für Aluminium im Bereich von 0,1 bis 1 mg/l und/oder bei einem Füllvolumen von 1 ml die Konzentration des ausgelaugten Bestandteiles für Silizium im Bereich von 3 bis 6mg/l , für Bor im Bereich von 0,4 bis 1 mg/l, für Natrium im Bereich von 1,5 bis 2,5 mg/l für Calcium im Bereich von 0,05 bis 0,3 mg/l und/oder für Aluminium im Bereich von 0,1 bis 0,7 mg/ml liegt.

16. Glasfläschchen (1) gemäß einem der Ansprüche 11 bis 15, wobei die Oberfläche der Innenwand (2) keine Beschichtung aufweist und/oder nicht geätzt wurde und/oder das Glas der Innenwandung (21) des Glasfläschchens (1) bis zu einer Tiefe von zumindest 200 nm einphasig ist.

17. Glasfläschchen (1) gemäß einem der vorstehenden Ansprüche 11 bis 16, wobei der Befüllungsgrad maximal 0,125 beträgt und/oder das Glasfläschchen (1) ein Nennvolumen im Bereich von 1 bis 2 ml aufweist.

18. Medizinprodukt, umfassend ein mit einer flüssigen pharmazeutischen Wirkstoffzubereitung (4) befülltes und verschlossenes Glasfläschchen (1) gemäß einem der vorherigen Ansprüche 11 bis 17.

## Claims

1. Use of a glass vial (1) having a total volume of < 4.5 ml, made of a multicomponent glass, wherein the inner wall (21) of the glass vial (1) has a chemical resistance to the leaching of at least one of the constituent parts of the multicomponent glass, wherein
in case of leaching of the glass vial (1) with an aqueous liquid having a pH value in the range of 5 to 9 at a temperature of 40°C over a period of 24 weeks when the glass vial is stored standing upright, the ratio of the concentration of at least one leached-out constituent part at a fill volume of 0.5 ml to the concentration at a fill volume of 2 ml is a maximum of 3, and the ratio between the concentration at a fill volume of 1 ml and the concentration at a fill volume of 2 ml is a maximum of 2,
for filling with a liquid pharmaceutical preparation (4) up to a filling level of a maximum of 0.25.

2. Use of a glass vial (1) according to claim 1, wherein in case of leaching of the glass vial (1) with an aqueous liquid having a pH value in the range of 5 to 9 at a temperature of 40°C over a period of 24 weeks when the glass vial is stored standing upright, the ratio of the concentration of the leached constituent part at a fill volume of 0.5 ml to the concentration at a fill volume of 2 ml is a maximum of 2.5, particularly preferably a maximum of 1.5, and/or the ratio between the concentration at a fill volume of 1 ml and the concentration at a fill volume of 2 ml is a maximum of 1.5, and/or the ratio between the concentration of the leached-out constituent part at a fill volume of 0.5 ml and the concentration at a fill volume of 1 ml is a maximum of 2.5, preferably a maximum of 1.7.

3. Use of a glass vial (1) according to claim 1, wherein purified water, 10% KCI solution, 0.5% NaCl solution, a phosphate buffer or an NaHCOs solution, preferably purified water, is used as the liquid for leaching the constituent parts of the glass.

4. Use of a glass vial (1) according to any of the preceding claims, wherein the glass vial (1) consists of a multicomponent glass comprising at least Si and Na, preferably comprising Si and Na and at least one of the constituent parts from the group made up of B, Al and/or Ca, and/or the multicomponent glass is a borosilicate glass, preferably a Type 1 neutral glass.

5. Use of a glass vial (1) according to any of the preceding claims, wherein in case of leaching of the glass vial (1) with purified water at a temperature of 40°C and a storage time of 24 weeks when the glass vial is stored upright, the ratio between the concentration of the leached constituent part at a fill volume of 1 ml and the concentration of the leached-out constituent part at a fill volume of 2 ml is a maximum of 1.5 for silicon, a maximum of 1.6 for sodium and/or a maximum of 2 for boron.

6. Use of a glass vial (1) according to the preceding claims, wherein in case of leaching of the glass vial (1) with an aqueous liquid having a pH value in the range of 5 to 9 the leaching intensity for the leached constituent part at a fill volume of 0.5 ml is less than the leaching intensity for the leached constituent part at a fill volume of 2 ml.

7. Use of a glass vial (1) according to any of the preceding claims, wherein in case of leaching of the glass vial (1) with 15% KCI solution at a temperature of 40°C and a storage time of 24 weeks when the glass vial is stored upright, at a fill volume of 0.5 ml the concentration of the leached-out constituent part is in the range of 1 to 3 mg/l for silicon, in the range of 0.2 to 1.2 mg/l for boron, in the range of 1.8 to 3.5 mg/l for sodium and/or in the range of 0.2 to 1 mg/l for calcium, and/or wherein in case of leaching of the glass vial (1) with 0.9% NaCl solution water at a temperature of 40°C and a storage time of 24 weeks when the glass vial is stored upright, at a fill volume of 0.5 ml the concentration of the leached constituent part is in the range of 2 to 4 mg/l for silicon, in the range of 0.6 to 1.5 mg/l for boron and/or in the range of 0.2 to 1 mg/l for calcium.

8. Use of a glass vial (1) according to any of the preceding claims, wherein in case of leaching of the glass vial (1) with 0.9% NaCl solution at a temperature of 40°C and a storage time of 24 weeks when the glass vial is stored upright, the ratio between the concentration of the leached constituent part at a fill volume of 0.5 ml and the concentration of the leached constituent part at a fill volume of 2 ml is a maximum of 2.5, preferably a maximum of 1.5, and/or the ratio between the concentration of the leached constituent part at a fill volume of 1 ml and the concentration of the leached constituent part at a fill volume of 2 ml is a maximum of 1.6 for silicon, preferably a maximum of 1.5.

9. Use of a glass vial (1) according to any of the preceding claims, wherein the liquid pharmaceutical active-substance preparation (4) contains therapeutic proteins, monoclonal antibodies and/or vaccines.

10. Use of a glass vial (1) according to any of the preceding claims, wherein the glass vial (1) can be produced using a method comprising at least the following steps:
- locally heating one end of a glass tube,
- separating the locally heated end of the glass tube to form a glass vial having a closed base, and
- further forming the base (3) of the glass vial (1), wherein:
- the formed glass vial (1); and
- in case of further forming the base of the glass vial with the help of a purge gas, a flow of purge gas (50) is generated inside the glass vial.

11. Glass vial (1) made of a multicomponent glass containing boron, with a liquid aqueous pharmaceutical active-substance preparation (4) having a pH value in the range of 5 to 9 and a sterile closure, wherein the glass vial (1) has a total volume of < 4.5 ml, the filling level of the glass vial (1) with the pharmaceutical active-substance preparation (4) is a maximum of 0.25 and wherein the inner wall (21) of the glass vial (1) has a chemical resistance to the leaching of at least one of the constituent parts of the multicomponent glass, wherein the ratio of the concentration of at least one leached constituent part at a fill volume of 0.5 ml to the concentration at a fill volume of 2 ml is a maximum of 3 and the ratio between the concentration at a fill volume of 1 ml and the concentration at a fill volume of 2 ml is a maximum of 2.

12. Glass vial (1) according to claim 11, wherein in case of leaching of the glass vial (1) with a liquid the ratio between the concentration of the leached-out constituent part at a fill volume of 0.5 ml and the concentration at a fill volume of 1 ml is a maximum of 2.5, preferably a maximum of 1.7.

13. Glass vial (1) according to any of the preceding claims 11 or 12, wherein the glass vial (1) consists of a multicomponent glass comprising Si and Na, preferably comprising Si, Na and at least one of the constituent parts from the group made up of B, Al and/or Ca, preferably of a borosilicate glass and particularly preferably of a neutral glass.

14. Glass vial (1) according to any of the preceding claims 11 to 13, wherein in case of leaching of the glass vial (1) with water, preferably with purified water, at a temperature of 40°C and a storage time of 24 weeks when the glass vial is stored upright, the ratio between the concentration of the leached constituent part at a fill volume of 0.5 ml and the concentration of the leached constituent part at a fill volume of 1 ml is a maximum of 1.5 for silicon, a maximum of 2.5 for sodium and/or a maximum of 3 for boron, and/or the ratio between the concentration of the leached constituent part at a fill volume of 1 ml and the concentration of the leached constituent part at a fill volume of 2 ml is a maximum of 1.5 for silicon, a maximum of 1.6 for sodium and/or a maximum of 2 for boron.

15. Glass vial (1) according to any of the preceding claims 11 to 14, wherein in case of leaching of the glass vial (1) with water at a temperature of 40°C and a storage time of 24 weeks when the glass vial is stored upright, at a fill volume of 0.5 ml the concentration of the leached constituent part is in the range of 3 to 6 mg/l for silicon, in the range of 0.8 to 1.6 mg/l for boron, in the range of 1.6 to 4 mg/l for sodium, in the range of 0.05 to 0.5 mg/l for calcium and/or in the range of 0.1 to 1 mg/l for aluminium, and/or at a fill volume of 1 ml the concentration of the leached-out constituent part is in the range of 3 to 6 mg/l for silicon, in the range of 0.4 to 1 mg/l for boron, in the range of 1.5 to 2.5 mg/l for sodium, in the range of 0.05 to 0.3 mg/l for calcium and/or in the range of 0.1 to 0.7 mg/ml for aluminium.

16. Glass vial (1) according to any of claims 11 to 15, wherein the surface of the inner wall (2) has no coating and/or has not been etched and/or the glass of the inner wall (21) of the glass vial (1) is monophasic to a depth of at least 200 nm.

17. Glass vial (1) according to any of the preceding claims 11 to 16, wherein the filling level is a maximum of 0.125 and/or the glass vial (1) has a nominal volume in the range of 1 to 2 ml.

18. Medical device comprising a glass vial (1) according to any of the preceding claims 11 to 17, which has been filled with a liquid pharmaceutical active-substance preparation (4) and sealed.

## Revendications

1. Utilisation d'un flacon en verre (1) avec un volume total < 4,5 ml composé d'un verre à plusieurs composants, dans laquelle la paroi interne (21) du flacon en verre (1) présente une résistance chimique empêchant une dissolution d'au moins un des constituants du verre à plusieurs composants, dans laquelle
lors de la dissolution du flacon en verre (1) avec un liquide aqueux avec une valeur pH dans la plage de 5 à 9 à une température de 40 °C sur une période de 24 semaines lors d'un stockage vertical du flacon en verre, le rapport de la concentration d'au moins un constituant lixivié pour un volume de remplissage de 0,5 ml et de la concentration pour un volume de remplissage 2 ml est de 3 au maximum et le rapport entre la concentration pour un volume de remplissage de 1 ml et la concentration pour un volume de remplissage de 2 ml est de 2 au maximum,
destiné à être rempli d'une préparation pharmaceutique liquide (4) jusqu'à un degré de remplissage de 0,25 au maximum.

2. Utilisation d'un flacon en verre (1) selon la revendication 1, dans laquelle lors de la dissolution du flacon en verre (1) avec un liquide aqueux avec une valeur pH dans la plage de 5 à 9 à une température de 40 °C sur une période de 24 semaines lors du stockage vertical du flacon en verre, le rapport de la concentration du constituant dissous pour un volume de remplissage de 0,5 ml et de la concentration pour un volume de remplissage de 2 ml est de 2,5 au maximum, de manière particulièrement préférée de 1,5 au maximum, et/ou le rapport entre la concentration pour un volume de remplissage de 1 ml et la concentration pour un volume de remplissage de 2 ml est de 1,5 au maximum et/ou le rapport entre la concentration du constituant lixivié pour un volume de remplissage de 0,5 ml et la concentration pour un volume de remplissage de 1 ml est de 2,5 au maximum, de manière préférée de 1,7 au maximum.

3. Utilisation d'un flacon en verre (1) selon la revendication 1, dans laquelle sont utilisés en tant que liquide pour dissoudre les constituants de verre de l'eau préparée, une solution de KCl à 10 %, une solution de NaCl à 0,5 %, un tampon phosphate ou une solution de NaHCOs, de manière préférée de l'eau préparée.

4. Utilisation d'un flacon en verre (1) selon l'une quelconque des revendications précédentes, dans laquelle le flacon en verre (1) est constitué d'un verre à plusieurs composants comprenant au moins du Si et du Na, comprenant de manière préférée du Si et du Na et au moins un des constituants issus du groupe formé par B, Al et/ou Ca, et/ou que le verre à plusieurs composants est un verre de borosilicate, de manière préférée un verre neutre de la classe I.

5. Utilisation d'un flacon en verre (1) selon l'une quelconque des revendications précédentes, dans laquelle lors de la dissolution du flacon en verre (1) avec de l'eau préparée à une température de 40 °C et pendant une durée de stockage de 24 semaines lors d'un stockage vertical du flacon en verre, le rapport entre la concentration du constituant dissous pour un volume de remplissage de 1 ml et la concentration du constituant lixivié pour un volume de remplissage de 2 ml est de 1,5 au maximum pour du silicium, de 1,6 au maximum pour du sodium, et/ou de 2 au maximum pour du bore.

6. Utilisation d'un flacon en verre (1) selon les revendications précédentes, dans laquelle la dissolution du flacon en verre (1) avec un liquide aqueux avec une valeur pH dans la plage de 5 à 9, l'intensité de dissolution pour le constituant dissous pour un volume de remplissage de 0,5 ml est inférieure à l'intensité de dissolution pour le constituant dissous pour un volume de remplissage de 2 ml.

7. Utilisation d'un flacon en verre (1) selon l'une quelconque des revendications précédentes, dans laquelle lors de la dissolution du flacon en verre (1) avec une solution de KCl à 15 % à une température de 40 °C et pendant une durée de stockage de 24 semaines lors d'un stockage vertical du flacon en verre pour un volume de remplissage de 0,5 ml, la concentration du constituant lixivié se situe pour dans la plage de 1 à 3 mg/l pour le silicium, dans la plage de 0,2 à 1,2 mg/l pour le bore, dans la plage de 1,8 à 3,5 mg/l pour le sodium et/ou dans la plage de 0,2 à 1 mg/l pour le calcium et/ou dans laquelle lors de la dissolution du flacon en verre (1) avec une solution de NaCl à 0,9 % à une température de 40 °C et pendant une durée de stockage de 24 semaines lors d'un stockage vertical du flacon en verre pour un volume de remplissage de 0,5 ml, la concentration du constituant dissous se situe dans la plage de 2 à 4 mg/l pour le silicium, dans la plage de 0,6 à 1,5 mg/l pour le bore et/ou dans la plage de 0,2 à 1 mg/l pour le calcium.

8. Utilisation d'un flacon en verre (1) selon l'une quelconque des revendications précédentes, dans laquelle lors de la dissolution du flacon en verre (1) avec une solution de NaCl à 0,9 % à une température de 40 °C et pendant une durée de stockage de 24 semaines lors d'un stockage vertical du flacon en verre, le rapport entre la concentration du constituant dissous pour un volume de remplissage de 0,5 ml et la concentration du constituant dissous pour un volume de remplissage de 2 ml est de 2,5 au maximum, de manière préférée de 1,5 au maximum, et/ou le rapport entre la concentration du constituant dissous pour un volume de remplissage de 1 ml et la concentration du constituant dissous pour un volume de remplissage de 2 ml est de 1,6 au maximum pour le silicium, de manière préférée de 1,5 au maximum.

9. Utilisation d'un flacon en verre (1) selon l'une quelconque des revendications précédentes, dans laquelle la préparation de principes actifs pharmaceutique liquide (4) contient des protéines thérapeutiques, des anticorps monoclonaux et/ou des vaccins.

10. Utilisation d'un flacon en verre (1) selon l'une quelconque des revendications précédentes, dans laquelle le flacon en verre (1) peut être fabriqué avec un procédé comprend au moins les étapes suivantes :
- le chauffage local d'une extrémité d'un tube en verre,
- la séparation de l'extrémité localement chauffée du tube en verre en réalisant un flacon en verre avec un fond fermé, et
- la mise en forme ultérieure du fond (3) du flacon en verre (1), dans laquelle :
- le flacon en verre (1) réalisé ; et
- lors de la mise en forme ultérieure du fond du flacon en verre à l'aide d'un gaz de rinçage, un écoulement de gaz de rinçage (50) est généré à l'intérieur des flacons en verre.

11. Flacon en verre (1) composé d'un verre à plusieurs composants contenant du bore avec une préparation de principes actifs pharmaceutique aqueuse (4) avec une valeur pH dans la plage de 5 à 9 et une fermeture stérile, dans lequel le flacon en verre (1) présente un volume total < 4,5 ml, le degré de remplissage du flacon en verre (1) avec la préparation de principes actifs pharmaceutique (4) est de 0,25 au maximum, et dans lequel la paroi interne (21) du flacon en verre (1) présente une résistance chimique empêchant une dissolution d'au moins un des constituants du verre à plusieurs composants, dans lequel le rapport de la concentration d'au moins un constituant dissous pour un volume de remplissage de 0,5 ml et la concentration pour un volume de remplissage de 2 ml est de 3 au maximum et le rapport entre la concentration pour un volume de remplissage de 1 ml et la concentration pour un volume de remplissage de 2 ml est de 2 au maximum.

12. Flacon en verre (1) selon la revendication 11, dans lequel lors de la dissolution du flacon en verre (1) avec un liquide, le rapport entre la concentration du constituant lixivié pour un volume de remplissage de 0,5 ml et la concentration pour un volume de remplissage de 1 ml est de 2,5 au maximum, de manière préférée de 1,7 au maximum.

13. Flacon en verre (1) selon l'une quelconque des revendications précédentes 11 ou 12, dans lequel le flacon en verre (1) est constitué d'un verre à plusieurs composants comprenant du Si et du Na, comprenant de manière préférée du Si, du Na et au moins un des constituants issus du groupe formé par B, Al, et/ou Ca, de manière préférée d'un verre de borosilicate et de manière particulièrement préférée d'un verre neutre.

14. Flacon en verre (1) selon l'une quelconque des revendications précédentes 11 à 13, dans lequel lors de la dissolution du flacon en verre (1) avec de l'eau, de manière préférée avec de l'eau préparée, à une température de 40 °C et pendant une durée de stockage de 24 semaines lors d'un stockage vertical du flacon en verre, le rapport entre la concentration du constituant dissous pour un volume de remplissage de 0,5 ml et la concentration du constituant dissous pour un volume de remplissage de 1 ml est de 1,5 au maximum pour le silicium, de 2,5 au maximum pour le sodium et/ou de 3 au maximum pour le bore, et/ou le rapport entre la concentration du constituant dissous pour un volume de remplissage de 1 ml et la concentration du constituant dissous pour un volume de remplissage de 2 ml est de 1,5 au maximum pour le silicium, de 1,6 au maximum pour le sodium et/ou de 2 au maximum pour le bore.

15. Flacon en verre (1) selon l'une quelconque des revendications précédentes 11 à 14, dans lequel lors de la dissolution du flacon en verre (1) avec de l'eau à une température de 40 °C et pendant une durée de stockage de 24 semaines lors d'un stockage vertical du flacon en verre pour un volume de remplissage de 0,5 ml, la concentration du constituant dissous se situe dans la plage de 3 à 6 mg/l pour le silicium, dans la plage de 0,8 à 1,6 mg/l pour le bore, dans la plage de 1,6 à 4 mg/l pour le sodium, dans la plage de 0,05 à 0,5 mg/l pour le calcium, et/ou dans la plage de 0,1 à 1 mg/l pour l'aluminium et/ou pour un volume de remplissage de 1 ml, la concentration du constituant lixivié se situe dans la plage de 3 à 6 mg/l pour le silicium, dans la plage de 0,4 à 1 mg/l pour le bore, dans la plage de 1,5 à 2,5 mg/l pour le sodium, dans la plage de 0,05 à 0,3 mg/l pour le calcium et/ou dans la plage de 0,1 à 0,7 mg/ml pour l'aluminium.

16. Flacon en verre (1) selon l'une quelconque des revendications 11 à 15, dans lequel la surface de la paroi interne (2) ne présente aucun revêtement, et/ou n'a pas été décapée, et/ou le verre de la cloison interne (21) du flacon en verre (1) est monophasé jusqu'à une profondeur d'au moins 200 nm.

17. Flacon en verre (1) selon l'une quelconque des revendications 11 à 16, dans lequel le degré de remplissage est de 0,125 au maximum et/ou le flacon de verre (1) présente un volume nominal dans la plage de 1 à 2 ml.

18. Produit médical comprenant un flacon en verre (1) selon l'une quelconque des revendications 11 à 17 fermé et rempli d'une préparation de principes actifs pharmaceutique liquide (4).
